# EUROPEAN PATENT APPLICATION

(11) **EP 4 802 991 A1**
(43) Date of publication of application: **09.09.2026**
(21) Application number: 23957089.8
(22) Date of filing: 31.10.2023
(51) Int. Cl.: A61B 5/00

(54) **PARAMETER CONNECTION COMPONENT, CIRCUIT BOARD STRUCTURE, PARAMETER ACQUISITION ASSEMBLY, AND DEVICES**

(71) Applicant: Shenzhen Mindray Scientific Co., Ltd., Shenzhen, Guangdong 518132 (CN)
(72) Inventor: LIU, Kuangpeng, Shenzhen, Guangdong 518132 (CN); ZHENG, Rongmin, Shenzhen, Guangdong 518132 (CN); YANG, Kuncai, Shenzhen, Guangdong 518132 (CN)
(74) Representative: KIPA AB
(86) International application number: PCT/CN2023/128256
(87) International publication number: WO 2025/091209

(57) **Abstract**

Disclosed are a parameter connection component (200), a circuit board structure, a parameter acquisition assembly, a monitoring auxiliary device (100), and a medical device. At least two parameter modules (1213) are provided within at least one parameter area (1201) of the circuit board (120), the parameter modules (1213) within the parameter area (1201) are electrically connected to a corresponding parameter interface (130), and the number of parameter interfaces (130) connected to at least one parameter area (1201) that is provided with at least two parameter modules (1213) is less than the number of parameter modules (1213) within the corresponding parameter area (1201). This allows at least two parameter modules (1213) within at least one parameter area (1201) that is provided with at least two parameter modules (1213) to be electrically connected to one parameter interface (130), and to be electrically connected to external parameter accessories separately via the parameter interface (130). As such, the number of parameter interfaces (130) of the monitoring auxiliary device (100) can be reduced without changing the number of physiological parameters monitored by the monitoring auxiliary device (100) or the number of external parameter accessories electrically connected to the monitoring auxiliary device (100), thereby allowing the monitoring auxiliary device (100) to be more compact.

## Description

### TECHNICAL FIELD

The disclosure relates to the technical field of medical instruments, and more particularly to a parameter connection component, a circuit board structure, a parameter acquisition assembly, a monitoring auxiliary device, and a medical device.

### BACKGROUND

Monitoring device is the most widely used medical device, and is usually arranged with multiple parameter interfaces, wherein each parameter interface is in electrical connection with different parameter accessories. Different parameter accessories can detect different physiological parameters, such as electrocardiogram, blood oxygen, body temperature, blood pressure, etc., of a patient, and can transmit these physiological parameters to the monitoring device, so as to monitor the physiological parameters, such as electrocardiogram, blood oxygen, body temperature, and blood pressure of the patient by the monitoring device, thus facilitating medical staff to know a physical sign state of the patient in real time.

A morphology of the monitoring device is varied, and as application scenarios are complicated and care requirements are improved, the morphology of the monitoring device is not limited to a traditional bedside monitor. Some forms of monitoring device have a miniaturization requirement. However, as the number of physiological parameters monitored by the monitoring device is increased, the number of parameter interfaces on the monitoring device is also increased, which is not conducive to the miniaturization of the monitoring device.

### SUMMARY

Embodiments of this disclosure provide a parameter connection component, a circuit board structure, a parameter acquisition assembly, a monitoring auxiliary device, and a medical device, aiming at a problem of difficulty in miniaturization of a monitoring auxiliary device, which problem has resulted by an increased number of physiological parameters monitored by the monitoring auxiliary device.

According to a first aspect, an embodiment of this disclosure provides a monitoring auxiliary device, including:
a housing, which includes an inner surface and an outer surface, wherein the inner surface encloses a mounting cavity;
a circuit board, which is mounted inside the mounting cavity, wherein the circuit board includes multiple parameter areas that are electrically isolated from each other; wherein at least one parameter module is arranged inside each parameter area of the multiple parameter areas; at least two parameter modules are arranged inside at least one parameter area of the multiple parameter areas; wherein each parameter module is configured to acquire a physiological parameter;
multiple parameter interfaces, wherein each parameter area of the multiple parameter areas is correspondingly connected with at least one parameter interface of the multiple parameter interfaces, each parameter module is in electrical connection with at least one parameter interface of the multiple parameter interfaces; wherein a number of parameter interface(s), which is(are) connected with at least one parameter area, is less than a number of parameter modules that are arranged inside said at least one parameter area, wherein at least two parameter modules are arranged inside said at least one parameter area; wherein each parameter area of the multiple parameter areas is capable of being in electrical connection with at least one external parameter accessory; and
a data output interface, which is at least partially mounted inside the mounting cavity and capable of being connected with a monitor through a cable, so as to output to the monitor, in a wired manner, the acquired physiological parameters and/or data that are generated based on processing the acquired physiological parameters.

According to a second aspect, an embodiment of this disclosure further provides a monitoring auxiliary device, including:
a housing, which includes an inner surface and an outer surface, wherein the inner surface encloses a mounting cavity;
a circuit board, which is mounted inside the mounting cavity, wherein the circuit board includes two or more parameter areas, wherein at least one parameter module is arranged inside each parameter area of the two or more parameter areas; the at least one parameter module is configured to acquire a physiological parameter; and
two or more parameter interfaces, wherein each parameter interface of the two or more parameter interfaces is in electrical connection with the at least one parameter module, a number of parameter interfaces is less than a number of parameter modules, wherein each parameter interface of the two or more parameter interfaces is capable of being in electrical connection with at least one external parameter accessory.

According to a third aspect, an embodiment of this disclosure further provides a circuit board structure, including:
a circuit board, which includes two or more parameter areas, wherein at least one parameter module is arranged inside each parameter area of the two or more parameter areas; the at least one parameter module is configured to acquire a physiological parameter; and
two or more parameter interfaces, wherein each parameter interface of the two or more parameter interfaces is in electrical connection with the at least one parameter module, a number of parameter interfaces is less than a number of parameter modules, wherein each parameter interface of the two or more parameter interfaces is capable of being in electrical connection with at least one external parameter accessory.

According to a fourth aspect, an embodiment of this disclosure further provides a medical device, including:
a housing, which includes an inner surface and an outer surface, wherein the inner surface encloses a mounting cavity;
a circuit board, which includes two or more parameter areas, wherein at least one parameter module is arranged inside each parameter area of the two or more parameter areas; the at least one parameter module is configured to acquire a physiological parameter; and
two or more parameter interfaces, wherein each parameter interface of the two or more parameter interfaces is in electrical connection with the at least one parameter module, a number of parameter interfaces is less than a number of parameter modules, wherein each parameter interface of the two or more parameter interfaces is capable of being in electrical connection with the at least one external parameter accessory.

According to a fifth aspect, an embodiment of this disclosure further provides a parameter connection component, which is configured to electrically connect a parameter accessory to a monitoring auxiliary device, wherein the monitoring auxiliary device includes a parameter interface and at least two parameter modules that are in electrical connection with the parameter interface, wherein the at least two parameter modules are configured to acquire a physiological parameter;
wherein the parameter connection component further includes a connector and at least two sub-connectors that are in electrical connection with the connector, wherein each sub-connector of the at least two sub-connectors is in electrical connection with at least one parameter accessory, wherein the connector is in electrical connection with the parameter interface, so as to electrically connect at least two parameter accessories to the at least two parameter modules in one-to-one correspondence.

According to a sixth aspect, an embodiment of this disclosure further provides a parameter acquisition assembly, which includes a connector and at least two parameter accessories of different types of physiological parameters, wherein each parameter accessory of the at least two parameter accessories is in respective electrical connection with the connector, the connector is further in electrical connection with a parameter interface of a monitoring auxiliary device, so as to transmit the physiological parameters acquired by the at least two parameter accessories to the monitoring auxiliary device.

The monitoring auxiliary device provided in embodiments of this disclosure arranges at least two parameter modules inside at least one parameter area of a circuit board, so as to enable the parameter modules inside the parameter area to be in electrical connection with corresponding parameter interface(s), wherein the number of parameter interface(s), which is(are)connected with the at least one parameter area that is arranged with the at least two parameter modules, is less than a corresponding number of parameter modules in said parameter area, such that the at least two parameter modules of said at least one parameter area that is arranged with said at least two parameter modules are in electrical connection with one parameter interface, and are in respective electrical connections with external parameter accessories through said one parameter interface. In such a way, the number of parameter interface(s) of the monitoring auxiliary device can be reduced, while maintaining the number of physiological parameters monitored by the monitoring auxiliary device unchanged or maintaining the number of external parameter accessories in electrical connection with the monitoring auxiliary device unchanged, thus miniaturizing the monitoring auxiliary device more.

### BRIEF DESCRIPTION OF THE DRAWINGS

Technical solutions and other beneficial effects of this disclosure will be apparent from following detailed description of specific embodiments of this disclosure with reference to accompanying drawings.
FIG. 1 is a partial cross-sectional view for an embodiment of a monitoring auxiliary device according to an embodiment of this disclosure.
FIG. 2 is a structural diagram for an embodiment of a circuit board and a parameter interface according to an embodiment of this disclosure.
FIG. 3 is a structural diagram for a monitoring auxiliary device and a connector of a first embodiment according to an embodiment of this disclosure.
FIG. 4 is a structural diagram for the connector in FIG. 3 after being connected with the monitoring auxiliary device.
FIG. 5 is a structural diagram for a monitoring auxiliary device and a connector of a second embodiment according to an embodiment of this disclosure.
FIG. 6 is a structural diagram for a monitoring auxiliary device and a connector of a third embodiment according to an embodiment of this disclosure.
FIG. 7 is a structural diagram for a monitoring auxiliary device and a connector of a fourth embodiment according to an embodiment of this disclosure.
FIG. 8 is a sectional view of an embodiment of a parameter interface and a connector according to an embodiment of this disclosure.
FIG. 9 is a sectional view of another embodiment of a parameter interface and a connector according to an embodiment of this disclosure.
FIG. 10 is a structural diagram for a parameter connection component according to an embodiment of this disclosure.
FIG. 11 is a structural diagram for another embodiment of a parameter connection component according to an embodiment of this disclosure.

Wherein: Monitoring auxiliary device 100; Housing 110; Inner surface 111; Mounting cavity 112; Outer surface 113; Opening 114; Opening edge 1141; Surrounding portion 115; Dividing plate 1151; Accommodating groove 1152; Sub-groove 1153; First mounting opening 1154; Second mounting opening 1155; Protrusion 116; Circuit board 120; Parameter area 1201; Insulated band 1202; Non-parameter area 1203; Isolated communication module 1211; Isolated power supply module 1212; Parameter module 1213; Data processing module 1214; Wireless communication module 1215; Pressure sensor 1216; Power management module 1217; Battery interface 1218; Charging interface 1219; Charging chip 1220; Indicator light 1221; Buzzer 1222; Pump interface 1223; Valve interface 1224; Data output interface 1225; Parameter interface 130; Connection pin 131; First pin 1311; Second pin 1312; Plug-in module 132; Parameter connection component 200; Connector 210; Housing body 211; Shielding portion 212; First connection element 213; Second connection element 214; Wiring harness 215; Wiring sub-harness 216; Wire adapter 217; Connection wiring harness 218; Sub-connector 219; First direction X.

### DESCRIPTION OF THE PREFERRED EMBODIMENTS

Technical solutions in embodiments of this disclosure are clearly and completely described as follows with reference to accompany drawings in embodiments of this disclosure. Obviously, described embodiments are only some embodiments of this disclosure, rather than not all embodiments of this disclosure. All other embodiments obtained by those skilled in the art based on the embodiments of this disclosure without creative labour shall fall within the protection scope of this disclosure.

In the description of this disclosure, it should be understood that terms, such as "centre", "longitudinal", "transverse", "length", "width", "thickness", "upper", "lower", "front", "rear", "left", "right", "vertical", "horizontal", "top", "bottom", "inner", "outer", "clockwise", "counterclockwise", etc., are based on orientation or positional relationship shown in drawings, and are only for convenience of describing this disclosure and simplifying description, rather than indicating or implying that indicated device or element must have a specific orientation, be constructed and operated in a specific orientation, and therefore cannot be understood as a limitation to this disclosure. In addition, terms "first" and "second" are only used for descriptive purposes, and cannot be understood as indicating or implying relative importance or implicitly indicating the number of indicated technical features. Thus, features defined with "first" and "second" may explicitly or implicitly include one or more of said features. In the description of this disclosure, "multiple" means two or more, unless otherwise expressly and specifically defined.

In the description of this disclosure, it is noted that, unless otherwise expressly and specifically defined, terms "mount", "connect to" and "connect with" should be understood in a broad sense, for example, a fixed connection, a detachable connection, or an integral connection; a mechanical connection, an electrical connection or a communicative connection between each other; a direct connection or an indirect connection through an intermediate medium, an internal communication between two elements or an interaction relationship between each other. For those skilled in the art, above terms in this disclosure can be understood according to specific situations.

In this disclosure, unless otherwise specifically specified and limited, a first feature "above" or "below" a second feature may include direct contact between the first and second features, or may include that first and second features are not in direct contact but in contact through another feature thereof. Similarly, a first feature being "above," "on top of" and "on" a second feature includes that the first feature is directly above and diagonally above the second feature, or simply means that a horizontal height of the first feature is higher that of the second feature. A first feature being "below," "under" and "underneath" a second feature includes that the first feature is directly under and diagonally under the second feature, or simply means that a horizontal height of the first feature is lower than the second feature.

The following disclosure provides different embodiments or examples for implementing different structures of this disclosure. To simplify the disclosure of this disclosure, components and arrangements of specific examples are described. Of course, they are merely examples and are not intended to limit this disclosure. In addition, this disclosure may repeat reference numerals and or reference letters in different embodiments or examples, and this repetition is for purposes of simplification and clarity and does not in itself indicate a relationship between various embodiments and or arrangements. In addition, this disclosure provides examples of various specific processes and materials, but those skilled in the art may be aware of use of other processes and/or materials.

Embodiments of this disclosure provide a parameter connection component, a circuit board structure, a parameter acquisition assembly, a monitoring auxiliary device, and a medical device. The following describes in detail below.

First, an embodiment of this disclosure provides a monitoring auxiliary device.

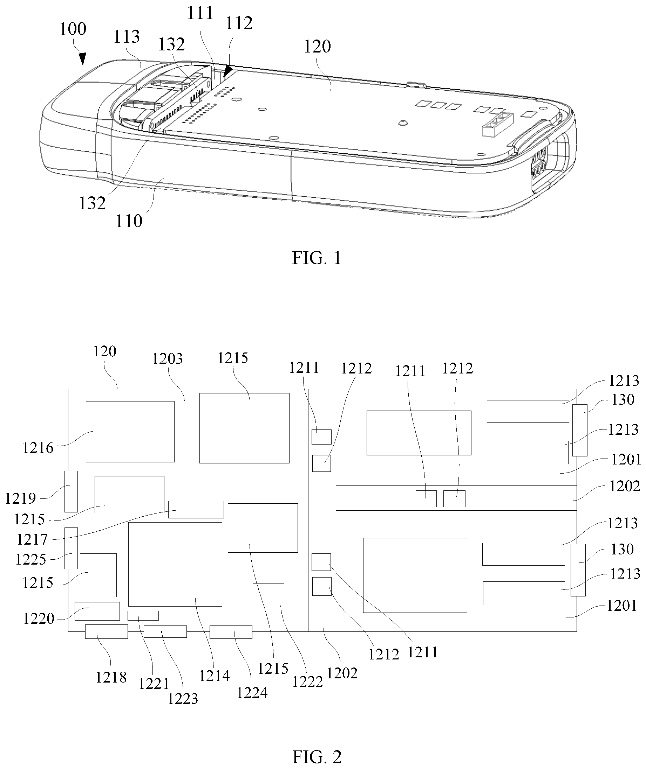

FIG. 1 is a partial cross-sectional view of an embodiment of a monitoring auxiliary device according to an embodiment of this disclosure. As shown in FIG. 1, the monitoring auxiliary device 100 includes a housing 110 and a circuit board 120 that is arranged inside the housing 110, wherein the circuit board 120 of the monitoring auxiliary device 100 is in electrical connection with an external parameter accessory (unshown). The parameter accessory is configured to detect physiological parameters, such as, blood oxygen, body temperature, and blood pressure of a patient. Different parameter accessories detect different physiological parameters of the patient. Accordingly, different physiological parameters, such as electrocardiogram, blood oxygen, body temperature, blood pressure and the likes of the patient, are detected by using different parameter accessories.

The circuit board 120 is configured to acquire a physiological parameter detected by a parameter accessory. The circuit board 120 of the monitoring auxiliary device 100 is in electrical connection with multiple different parameter accessories, so as to monitor different physiological parameters of a patient. For example, after the circuit board 120 of the monitoring auxiliary device 100 is in electrical connection with a parameter accessory for detecting an electrocardiogram parameter of a patient, the electrocardiogram parameter detected by said parameter accessory can be acquired, so as to monitor electrocardiogram of said patient; after the circuit board 120 of the monitoring auxiliary device 100 is in electrical connection with a parameter accessory for detecting a blood oxygen parameter of a patient, said patient blood oxygen parameter detected by said parameter accessory can be acquired, so as to monitor blood oxygen of said patient; after the circuit board 120 of the monitoring auxiliary device 100 is in electrical connection with a parameter accessory for detecting a blood pressure parameter of a patient, the blood pressure parameter of said patient detected by said parameter accessory can be acquired, so as to monitor blood pressure of said patient; after the circuit board 120 of the monitoring auxiliary device 100 is in electrical connection with a parameter accessory for detecting a body temperature parameter of a patient, the body temperature parameter detected by said parameter accessory can be acquired, so as to monitor body temperature of said patient.

The circuit board 120 of the monitoring auxiliary device 100 can be in electrical connection with one parameter accessory or can be in electrical connection with multiple parameter accessories. In addition, when the monitoring auxiliary device 100 is in electrical connection with multiple parameter accessories, the monitoring auxiliary device 100 may include multiple circuit boards 120, and each circuit board 120 is in respective electrical connection with different parameter accessories, so as to acquire different physiological parameters.

After acquiring parameters transmitted by various parameter accessories, the monitoring auxiliary device may collect these parameters, or then transmit the parameters to a monitor (such as a bedside monitor).

Continue referring to FIG. 1, the housing 110 includes an inner surface 111 and an outer surface 113, the inner surface 111 of the housing 110 encloses a mounting cavity 112, wherein the circuit board 120 is mounted inside the mounting cavity 112. As show in FIG. 2, at least one parameter module 1213 is arranged on the circuit board 120 for acquiring a physiological parameter. The parameter module 1213 on the circuit board 120 is in electrical connection with a parameter accessory, so as to acquire the physiological parameter.

When the circuit board 120 is arranged with multiple parameter modules 1213, the multiple parameter modules 1213 on the circuit board 120 may be in respective electrical connection with multiple parameter accessories, so as to enable different parameter modules 1213 to acquire different types of physiological parameters. Of course, the circuit board 120 may also be arranged with only one parameter module 1213, and the circuit board 120 is only in electrical connection with one parameter accessory, so as to acquire one type of physiological parameter.

In some embodiments, the parameter module 1213 includes a measurement module for a body temperature, a measurement module for an invasive blood pressure, a measurement module for electrocardiogram, a measurement module for blood oxygen, and the likes. The measurement module for a body temperature is configured to acquire a body temperature parameter of a patient. The measurement module for an invasive blood pressure is configured to acquire a blood pressure parameter of a patient. The measurement module for electrocardiogram is configured to acquire an ECG parameter of a patient. The measurement module for blood oxygen is configured to acquire a blood oxygen parameter of a patient.

In some embodiments, at least two parameter modules 1213 on the circuit board 120 are electrically isolated from each other, thereby avoiding a leakage current between the at least two parameter modules 1213 and satisfying a safety requirement.

As shown in FIG. 2, the circuit board 120 includes two or more parameter areas 1201, each parameter area 1201 is arranged inside with at least one parameter module 1213. In some embodiments, multiple parameter areas 1201 of the circuit board 120 are electrically isolated from each other, so as to isolate the parameter modules 1213 arranged inside different parameter areas 1201 from each other. When multiple parameter modules 1213 are arranged inside one parameter area 1201, said multiple parameter modules 1213 located in the same parameter area 1201 may be electrically isolated from each other, or may not be electrically isolated from each other.

In some embodiments, an insulated band 1202 may be arranged between two adjacent parameter areas 1201 of the circuit board 120, so as to electrically isolate the two adjacent parameter areas 1201. The insulated band 1202 may be an isolation area between the two adjacent parameter areas 1201 of the circuit board 120, a material of a portion of the circuit board 120, which portion is located inside the isolation area, is an insulation material, so as to achieve electrical isolation between the two adjacent parameter areas 1201 of the circuit board 120.

Of course, the circuit board 120 may also include multiple sub-boards, wherein two adjacent sub-boards are spaced apart from each other, or two adjacent sub-boards are connected by an insulation portion, a surface of each sub-board is one parameter area 1201, at least one parameter module 1213 may be arranged inside said one parameter area 1201, and the insulated band 1202 is formed by an area between two adjacent sub-boards, so as to electrically isolate the two adjacent sub-boards from each other. The multiple sub-boards of the circuit board 120 may be directly connected with the housing 110, or the multiple sub-boards of the circuit board 120 may be mounted on an isolation seat, and then the isolation seat is connected with the housing 110.

An insulated band 1202 is arranged between arbitrary two adjacent parameter areas 1201 of the circuit board 120, so as to electrically isolate the multiple parameter areas 1201 of the circuit board 120 from each other. The insulated band 1202 extends circumferentially along a parameter area 1201 to electrically isolate said parameter area 1201 from other parameter area(s) 1201 adjacent to each side of said parameter area 1201. As shown in FIG. 2, when one side of a parameter area 1201 is located at an edge of the circuit board 120, an insulated band 1202 extends along a circumferential direction of said parameter area to other three sides of said parameter area, and both ends of the insulated band 1202 are located at the edge of the circuit board 120.

In some embodiments, a minimum gap between parameter modules 1213 respectively located inside two adjacent parameter areas 1201 is greater than or equal to 5.2 mm. Each parameter module 1213 on the circuit board 120 has an orthographic projection on the circuit board 120, and a minimum distance between orthographic projections of the parameter modules 1213, which modules are respectively located inside the two adjacent parameter areas 1201 on the circuit board 120, is greater than or equal to 5.2 mm.

Therefore, the parameter modules 1213 inside two adjacent parameter areas 1201 can maintain a large gap, so as to enable the parameter modules 1213 inside the two adjacent parameter areas 1201 to have a good electrical isolation effect. The minimum gap between the parameter modules 1213 respectively located inside the two adjacent parameter areas 1201 may be 5.5 mm, 5.8 mm, 6 mm, 6.3 mm, etc., and may be specifically determined according to a structure of the circuit board 120 and type(s) of the parameter modules 1213 inside the parameter areas 1201.

It should be noted that, the minimum distance of the parameter modules 1213 inside arbitrary two adjacent parameter areas may be greater than or equal to 5.2 mm. Alternatively, a minimum distance between some parameter modules 1213 inside two adjacent parameter areas is greater than or equal to 5.2 mm, and a minimum distance of the other parameter modules 1213 inside other two adjacent parameter areas is less than 5.2 mm, which may be specifically determined according to a type of the parameter module 1213 inside each parameter area 1201.

In some embodiments, multiple parameter areas 1201 of the circuit board 120 are separately grounded, so as to electrically isolate the multiple parameter areas 1201 of the circuit board 120 from each other. Specifically, each parameter area 1201 of the circuit board 120 is arranged with a grounding portion, wherein grounding portions of each parameter area 1201 are electrically isolated from each other and are respectively grounded.

In some embodiments, an isolated communication module 1211 and/or an isolation power module 1212 are arranged between at least two adjacent parameter areas 1201 of the circuit board 120 for implementing data transmission between parameter modules 1213 within the two adjacent parameter areas 1201. The isolated communication module 1211 may be a magnetic coupler or an optocoupler, which is arranged on the insulated band 1202 of two adjacent parameter areas 1201. The two adjacent parameter areas 1201 may realize data transmission or communication and maintain an electrical isolation state of the two adjacent parameter areas 1201, by means of the magnetic coupler or the optocoupler.

The isolation power module 1212 is configured to implement power conversion between parameter modules 1213 within two adjacent parameter areas 1201. The isolated power supply module 1212 may be a transformer, which is arranged on the insulated band 1202 of two adjacent parameter areas 1201, and the parameter modules 1213 inside the two adjacent parameter areas 1201 can realize power conversion and maintain the electrical isolation state of two adjacent parameter areas 1201, by means of the transformer.

Parameter modules 1213 inside at least two adjacent parameter areas 1201 of the circuit board 120 are in electrical connection with the isolated communication module 1211 and/or the isolation power module 1212 between the two adjacent parameter areas 1201, so as to realize data transmission between parameter modules 1213 inside the two adjacent parameter areas 1201, and/or to implement power conversion between the parameter modules 1213 inside the two adjacent parameter areas 1201.

In an embodiment of this disclosure, multiple parameter modules 1213, which are arranged inside a same parameter area 1201 of the circuit board 120, have multiple types or combinations. For example, a measurement module for a body temperature of the monitoring auxiliary device 100 and a measurement module for an invasive blood pressure of the monitoring auxiliary device 100 are arranged inside a same parameter area 1201 of the circuit board 120; a measurement module for electrocardiogram of the monitoring auxiliary device 100 and a measurement module for blood oxygen of the monitoring auxiliary device 100 are arranged inside a same parameter area 1201 of the circuit board 120.

Continue referring to FIG. 2, the circuit board 120 further includes a non-parameter area 1203 adjacent to at least one parameter area 1201, wherein the non-parameter area 1203 is electrically isolated from the at least one parameter area 1201 that is adjacent to the non-parameter area 1203. Other functional modules other than the parameter module 1213 may be arranged inside the non-parameter area 1203.

Wherein an insulated band 1202 is arranged between the non-parameter area 1203 of the circuit board 120 and the at least one adjacent parameter area 1201 of the circuit board 120, so as to electrically isolate the non-parameter area 1203 from the at least one parameter area 1201 adjacent thereto. For example, a data processing module 1214 may be arranged inside the non-parameter area 1203 of the circuit board 120, wherein the data processing module 1214 is configured to receive a physiological parameter acquired by the parameter module 1213 or process and generate data based on the physiological parameter, so as to facilitate the monitoring auxiliary device 100 to display the physiological parameter or the data generated based on processing the physiological parameter.

In some embodiments, the monitoring auxiliary device 100 further includes a wireless communication module 1215 in electrical connection with the circuit board 120, wherein the wireless communication module 1215 is configured to transmit to a bedside monitor or another external device the physiological parameter acquired by the parameter module 1213 or the data generated based on processing the physiological parameter. The wireless communication module 1215 may be arranged inside a non-parametric area 1203 of the circuit board 120, and may be a Wireless Fidelity (Wi-Fi) module, a Near Field Communication (NFC) module, a Bluetooth Low Energy (BLE) module, a Wireless Medical Telemetry Service (WMTS) module, etc.

Of course, the non-parameter area 1203 of the circuit board 120 may also be arranged with one or more of: a pressure sensor 1216, a power management module 1217, a battery interface 1218 and a charging interface 1219. The pressure sensor 1216 is configured to acquire an air pressure signal in a cuff. The power management module 1217 may be configured to control functions, such as power-on and power-off of the whole monitoring auxiliary device 100, power-up sequence for respective battery domains inside the monitoring auxiliary device 100, and charging and discharging batteries inside the monitoring auxiliary device 100. The battery interface 1218 is connected with a battery, so as to realize power supply of the battery to the monitoring auxiliary device 100. The charging interface 1219 is connected with an external charging device (for example, an external power grid), so as to charge the monitoring auxiliary device 100 by the external charging device.

Furthermore, the non-parameter area 1203 of the circuit board 120 may also be arranged with a charging chip 1220, an indicator light 1221, a buzzer 1222, a pump interface 1223, a valve interface 1224, etc. The pump interface 1223, the valve interface 1224 and the battery interface 1218 are arranged on a same side edge of the circuit board 120.

It should be noted that, the non-parameter area 1203 of the circuit board 120 may be arranged with one or more of: a data processing module 1214, a pressure sensor 1216, a power management module 1217, a wireless communication module 1215, a battery interface 1218 and a charging interface 1219, a charging chip 1220, an indicator light 1221, a buzzer 1222, a pump interface 1223, a valve interface 1224, etc.

In some embodiments, an isolated communication module 1211 and/or an isolation power module 1212 are/is arranged between a non-parameter area 1203 and at least one parameter area 1201 adjacent to said non-parameter area 1203. The isolated communication module 1211 is configured to transmit to the data processing module 1214 a physiological parameter acquired by the parameter module 1213 of the parameter area 1201 or data generated based on processing the physiological parameter. The isolation power module 1212 is configured to implement a power conversion between the non-parameter area 1203 and the parameter area 1201.

An isolated communication module 1211 between a non-parameter area 1203 and an adjacent parameter area 1201 may be arranged on an insulated band 1202 between said non-parameter area 1203 and said adjacent parameter area 1201. An isolation power module 1212 between a non-parameter area 1203 and an adjacent parameter area 1201 may also be arranged on an insulated band 1202 between said non-parameter area 1203 and said adjacent parameter area 1201. A type of the isolated communication module 1211 and the isolated power supply module 1212, may refer to above embodiments, and not be repeated here.

As shown in FIG. 2, each parameter area 1201 may be adjacent to a non-parameter area 1203, respectively. The non-parameter area 1203 is electrically isolated from each parameter area 1201, respectively. An isolated communication module 1211 and/or an isolation power module 1212 are/is respectively arranged between the non-parameter area 1203 and said each parameter area 1201.

Wherein, at least one parameter module 1213 within a parameter area 1201 adjacent to a non-parameter area 1203 is in respective electrical connection with an isolated communication module 1211 and/or an isolation power module 1212 between the parameter area 1201 and the non-parameter area 1203, so as to achieve data transmission between the parameter module 1213 within the parameter area 1201 and the adjacent non-parameter area 1203, and/or to implement a power conversion between the parameter module 1213 within the parameter area 1201 and the adjacent non-parameter area 1203.

In some embodiments, the non-parameter area 1203 is arranged with a data processing module 1214, wherein the isolated communication module 1211 is configured to transmit to the data processing module 1214 a physiological parameter acquired by the parameter module 1213 of the parameter area 1201 or data generated based on processing the physiological parameter acquired by the parameter module 1213 of the parameter area 1201. The isolation power module 1212 is configured to implement a power conversion between the non-parameter area 1203 and the parameter area 1201.

In some embodiments, the monitoring auxiliary device 100 may be in communicative connection with a monitor through a cable, so as to enable the monitoring auxiliary device 100 to output to the monitor, in a wired manner, the acquired physiological parameter and/or data generated based on processing the acquired physiological parameter, wherein the monitor displays the physiological parameter and/or the data generated based on processing the physiological parameter.

It may be understood that the monitoring auxiliary device 100 may directly output the physiological parameter to the monitor, and the monitor displays the physiological parameter, or the monitor processes the physiological parameter and displays data generated based on processing the physiological parameter. The monitoring auxiliary device 100 may also process the physiological parameter, and output to the monitor data generated based on processing the physiological parameter, and the monitor displays the data generated based on processing the physiological parameter.

In some embodiments, the monitoring auxiliary device 100 includes a data output interface 1225, which is mounted at least partially within the mounting cavity 112, wherein the data output interface 1225 is connected with the monitor through a cable, so as to enable communication between the monitoring auxiliary device 100 and the monitor. The monitoring auxiliary device 100 outputs an acquired physiological parameter and/or data generated based on processing the physiological parameter to the monitor in a wired manner through the data output interface 1225.

In some embodiments, the data output interface 1225 may be connected with the data processing module 1214, wherein the data processing module 1214 is configured to receive a physiological parameter acquired by the parameter module 1213 or data generated based on processing the physiological parameter, and output to the monitor, through the data output interface 1225, the acquired physiological parameter and/or the data generated based on processing the physiological parameter.

The data output interface 1225 may be arranged on the circuit board 120, specifically for example: the data output interface 1225 is arranged inside a non-parameter area 1203 of the circuit board 120 and is in electrical connection with the data processing module 1214. Of course, the data output interface 1225 may be arranged on the housing 110 and in electrical connection with the data processing module 1214 on the circuit board 120 in a wired or wireless manner.

In other embodiments, the monitoring auxiliary device 100 may also be in wireless communicative connection with the monitor through a wireless communication module 1215, so as to realize data transmission between the monitoring auxiliary device 100 and the monitor, and enable the monitoring auxiliary device 100 to output to the monitor an acquired physiological parameter and/or data generated based on processing the physiological parameter.

In addition, the monitoring auxiliary device 100 may also be in wireless communicative connection with the monitor by means of a hard contact, so as to implement data transmission between the monitoring auxiliary device 100 and the monitor.

In some embodiments, the monitoring auxiliary device 100 may be a wearable device, a portable monitoring auxiliary device, a handheld monitoring auxiliary device, or the likes. Of course, the monitoring auxiliary device 100 may also be other types of monitoring auxiliary device, only if such monitoring auxiliary device satisfies limitation(s) of embodiments of this disclosure.

As shown in FIG. 2, the monitoring auxiliary device 100 further includes two or more parameter interfaces 130, wherein each parameter interface 130 is in electrical connection with at least one parameter module 1213, the number of parameter interfaces 130 is less than the number of parameter modules 1213, each parameter interface 130 is in electrical connection with at least one external parameter accessory.

In some embodiments, the monitoring auxiliary device 100 includes multiple parameter interfaces 130, each parameter area 1201 of the circuit board 120 is correspondingly connected with at least one parameter interface 130, so as to enable the parameter module 1213 within the parameter area 1201 to be in electrical connected with a corresponding parameter interface 130, i.e., so as to enable the parameter module 1213 within the parameter area 1201 to be in electrical connection with a corresponding parameter interface 130 that is connected with said parameter area 1201. Each parameter module 1213 is in electrical connection with at least one parameter interface 130. Each parameter interface 130 may be in electrical connection with at least one external parameter accessory, so as to electrically connect the parameter module 1213 that is located inside the parameter area 1201 of the circuit board 120 with an external parameter accessory. The parameter interface 130 may be at least partially mounted inside the mounting cavity 112 of the housing 110.

In some embodiments, the number of parameter interface(s) 130, which is(are) connected with at least one parameter area 1210 of the circuit board 120, is less than the number of parameter modules 1213 that are arranged inside said at least one parameter area 1210; wherein said at least one parameter area 1210 is a parameter area 1210 which is arranged inside with at least two parameter modules 1213. That is, the number of parameter interface(s) 130, which is(are) connected with at least one parameter area 1210 of the circuit board 120, is less than the number of parameter modules 1213 that are arranged inside a corresponding parameter area 1210.

The monitoring auxiliary device 100 provided in an embodiment of this disclosure arranges at least two parameter modules 1213 inside at least one parameter area 1201 of the circuit board 120, and enables the parameter modules 1213 inside the parameter areas 1201 to be in electrical connection with a corresponding parameter interface 130, wherein the number of parameter interface(s) 130, which is(are) connected with at least one parameter area 1201 that is arranged with the at least two parameter modules 1213, is less than the number of parameter modules 1213 in said at least one parameter area 1201, such that said at least two parameter modules 1213 of said at least one parameter area that is arranged with said at least two parameter modules are in electrical connection with one parameter interface 130, and are in respective electrical connection with external parameter accessories through said parameter interface 130. In such a way, the number of parameter interface(s) 130 of the monitoring auxiliary device 100 can be reduced, while maintaining the number of physiological parameters monitored by the monitoring auxiliary device 100 unchanged or maintaining the number of external parameter accessories in electrical connection with the monitoring auxiliary device unchanged, thus miniaturizing the monitoring auxiliary device more.

In particulate, when the parameter areas 1201 of the circuit board 120 are electrically isolated from each other, the parameter interface 130 can satisfy a safety requirement without providing a shielding structure, which can further reduce a size of the parameter interface 130 and satisfy a miniaturization requirement of the monitoring interface to a greatest extent.

It can be understood that the number of parameter interface(s) 130, which is(are) connected with each parameter area 1201 of the circuit board 120, may be less than the number of parameter modules 1213 in a corresponding parameter area 1201. The number of parameter interface(s) 130, which is(are) connected with one parameter area 1201 or some parameter areas 1201 of the circuit board 120, may be less than the number of parameter modules 1213 in corresponding parameter area(s) 1201, while the number of parameter interface(s) 130, which is(are) connected with other parameter area(s) 1201 of the circuit board 120, is equal to the number of parameter module(s) 1213 in corresponding parameter area(s) 1201.

Wherein, when the number of parameter interface(s) 130, which is(are) connected with each parameter area 1201 of the circuit board 120, is less than the number of parameter modules 1213 in a corresponding parameter area 1201, a difference between the number of parameter interface(s) 130 which is(are) connected with each parameter area 1201 of the circuit board 120, and the number of parameter modules 1213 in the corresponding parameter area 1201 may be 1, 2, 3, etc. The lager the difference between the number of parameter interface(s) 130 which is(are) connected with each parameter area 1201 of the circuit board 120, and the number of parameter modules 1213 in the corresponding parameter area 1201, the larger a reduced number of parameter interfaces 130 while maintaining the number of parameter modules 1213 unchanged, the greater a miniaturization of the monitoring auxiliary device.

When the number of the parameter interface(s) 130, which is(are) connected with multiple parameter areas 1201, is less than the number of parameter modules 1213 in corresponding parameter area(s) 1201, a difference between the number of the parameter interface(s) 130, which is(are) connected with the multiple parameter areas 1201, and the number of parameter modules 1213 in corresponding parameter area(s) 1201 can be the same or different, which may specifically be determined to according to respective numbers of parameter modules 1213 inside the multiple parameter areas 1201 and size(s) or type(s) of the parameter interface(s) 130, which is(are) connected with the multiple parameter areas 1201.

In some embodiments, parameter modules 1213, which are arranged inside at least one of parameter area(s) 1201 that is(are) arranged inside with at least two parameter modules 1213, are in electrical connection with one parameter interface 130. That is, multiple parameter modules 1213 inside at least one parameter area 1201 are in electrical connection with one same parameter interface 130, so as to reduce the number of parameter interface 130 which parameter interface 130 is correspondingly connected with the at least one parameter area 1201 to be one, wherein a difference between the number of parameter interface 130, which parameter interface 130 is connected with the parameter area 1201, and the number of parameter modules 1213 inside the corresponding parameter area 1201 reaches a maximum value, thereby reducing the number of parameter interface 130.

When, parameter modules 1213 inside each parameter area 1201 that is arranged inside with at least two parameter modules 1213 may be in electrical connection with one corresponding parameter interface 130. That is, multiple parameter areas 1201 of the circuit board 120, which parameter areas 1201 are arranged inside with at least two parameter modules 1213, are connected with multiple parameter interfaces 130 in one-to-one correspondence, and multiple parameter modules 1213 inside each parameter area 1201 are connected with one parameter interface 130 corresponding to said parameter area 1201, such that a total number of the parameter interfaces 130 is minimized, when parameter modules 1213 of each parameter area 1201 are electrically isolated from each other, so as to achieve a miniaturization of the monitoring auxiliary device 100 to a greatest extent.

Of course, a parameter module 1213 inside some parameter area(s) 1201 of the multiple parameter areas 1201 can be in electrical connection with one corresponding parameter interface 130, while the number of parameter interface(s) 130 that is(are) connected with the other parameter area(s) 1201 is less than or equal to the number of parameter modules 1213 in corresponding parameter area(s) 1201, such that the number of parameter interface(s) 130 can also be reduced to a certain extent.

One inventive point of this disclosure is that parameter interfaces of one certain parameter area or parameter interfaces of multiple parameter areas are combined. In a conventional technology, when how many parameter modules exist in a parameter area that is electrically isolated from other areas, how many corresponding interfaces exist. However, in this disclosure, the number of parameter interface(s) of one parameter area is less than the number of parameter modules in said parameter area, that is, at least one parameter area has combined interface(s). For example, there are two parameter modules but only one parameter interface in one parameter area, that is, the parameter area has a combined interface. For another example, there are two parameter areas and there are three parameter modules inside each parameter area, but each parameter area only corresponds to one or two parameter interfaces, or just one parameter area corresponds to one or two parameter interfaces and the other parameter area corresponds to three parameter interfaces, such that there is only one parameter area has a combined interface, and therefore, a feature of this solution can be summarized as: the number of parameter interface(s), which is(are) connected with at least one parameter area that is arranged inside with at least two parameter modules, is less than the number of parameter modules inside said at least one parameter area.

In some embodiments, as shown in FIG. 1, FIG. 2 and FIG. 8, at least one parameter area 1201 of the circuit board 120 is arranged inside with a plug-in module 132, wherein the plug-in module 132 inside the at least one parameter area 1201 is in electrical connection with a parameter module 1213, the parameter interface 130 is in connection with the plug-in module 132 via plug-in, so as to enable the parameter module 1213 inside the at least one parameter area 1201 of the circuit board 120 to be connected with a corresponding parameter interface 130, and to enable the parameter module 1213 inside the at least one parameter area 1201 to be in electrical connection with the corresponding parameter interface 130.

The plug-in module 132 may be welded or otherwise mounted to the parameter area 1201 of the circuit board 120, and the plug-in module 132 is in electrical connection with the parameter module 1213 by means of a conductive line arranged inside the parameter area 1201. The plug-in module 132 may be a board-to-board female connector (or board-to-board male connector), while the parameter interface 130 includes a board-to-board male connector (or board-to-board female connector), such that the parameter interface 130 may be in connection with the plug-in module 132 by plugging the board-to-board male connector with the board-to-board female connector. Of course, the parameter interface 130 and the plug-in module 132 may also implement plug-in connection in other ways, only if the parameter interface 130 be in electrical connection with the parameter module 1213 in a corresponding parameter area 1201 through the plug-in module 132.

It should be noted that one or more parameter areas 1201 of multiple parameter areas 1201 may be in plugged connection with a corresponding parameter interface 130 through a plug-in module 132, or all parameter areas 1201 may be in plugged connection with corresponding parameter interface(s) 130 through plug-in module(s) 132.

In some embodiments, at least one parameter interface 130 of the circuit board 120 may be mounted inside a corresponding parameter area 1201, so as to electrically connect the parameter interface 130 with parameter module(s) 1213 within a corresponding parameter area 1201. The parameter interface 130 may be mounted inside the corresponding parameter area 1201 by welding, pasting, etc. In addition, each parameter interface 130 may be mounted inside a corresponding parameter area 1201 of the circuit board 120, or some parameter interfaces 130 of multiple parameter interfaces 130 may be mounted inside corresponding parameter area(s) 1201.

In some embodiments, a parameter module 1213 of at least one parameter area 1201 of the circuit board 120 may be in electrical connection with a corresponding parameter interface 130 through a connection wire. For example, the parameter interface 130 may be in electrical connection with a circuit of a corresponding parameter area 1201 via a flexible flat cable (FFC), so as to enable the parameter interface 130 to be in electrical connection with the parameter module 1213 within a corresponding parameter area 1201. Each parameter interface 130 may be in electrical connection with a corresponding parameter module 1213 via a connection wire, or some of multiple parameter interfaces 130 may be in respective electrical connection with a corresponding parameter module 1213 via a connection wire.

It should be noted that the circuit board 120 can connect a parameter area 1201 with a corresponding parameter interface 130 according to above-mentioned connection manner, or different parameter areas 1201 of the circuit board 120 can also be connected with corresponding parameter interface(s) 130 according to two or three different ways.

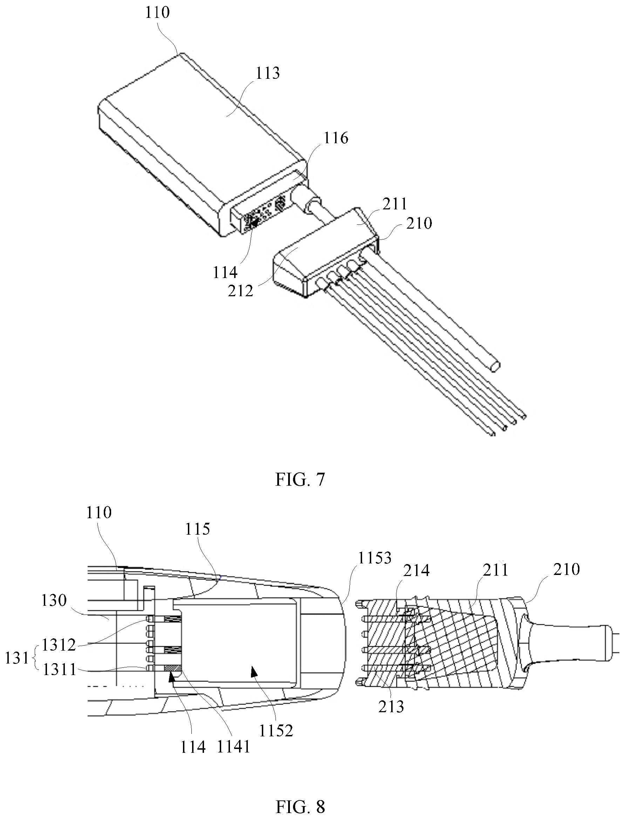

As shown in FIG. 8, an outer surface 113 of the housing 110 is arranged with multiple holes 114, which respectively extend to an inner surface 111 and connect with a mounting cavity 112, wherein each opening 114 enables at least one parameter interface 130 to be in electrical connection with an external parameter accessory.

Wherein, each opening 114 exposes at least one parameter interface 130, so as to enable the parameter interface 130 to be in electrical connection with an external parameter accessory. The opening 114 exposes the parameter interface 130 in multiple ways. The parameter interface 130 is entirely located inside the mounting cavity 112 of the housing 110 and faces the opening 114, so as to enable the opening 114 to expose the parameter interface 130, and a connector 210 in electrical connection with a parameter accessory can pass through the opening 114 and extend into the mounting cavity 112 to be connected with the parameter interface 130, so as to connect the parameter accessory with the corresponding parameter interface 130. Alternatively, a portion of the parameter interface 130 is located inside the mounting cavity 112 of the housing 110 and the other portion of the parameter interface 130 is located inside the opening 114, so as to enable the opening 114 to expose the parameter interface 130, and a connector 210 in electrical connection with a parameter accessory can be inserted into the opening 114 to be connected with the parameter interface 130, so as to connect the parameter accessory with the corresponding parameter interface 130. Alternatively, a portion of the parameter interface 130 is located inside the mounting cavity 112 of the housing 110, another portion of the parameter interface 130 passes through the opening 114 and extends out of the outer surface 113 of the housing 110, so as to enable the opening 114 to expose the parameter interface 130, and a connector 210 in electrical connection with a parameter accessory can be connected with the parameter interface 130 without being inserted into the opening 114.

As shown in FIG. 8, the opening 114 includes an opening edge 1141 located on the outer surface 113, the parameter interface 130 includes a connection pin 131 in electrical connection with a parameter accessory, wherein the connection pin 131 is located inside the mounting cavity 112 and/or the opening 114, so as to enable the housing 110 to provide a certain blocking for the connection pin 131 of the parameter interface 130, so as to prevent a patient from touching the connection pin 131 of the parameter interface 130.

In some embodiments, a minimum distance between a connection pin 131 of at least one parameter interface 130 and an opening edge 1141 of a corresponding opening 114 is less than 3 mm, so as to further reduce a size of the housing 110, which is beneficial to a miniaturization of the monitoring auxiliary device 100. A minimum distance between the connection pin 131 of the parameter interface 130 and the opening edge 1141 of the corresponding opening 114 may be 2.8 mm, 2.5 mm, 2 mm, 1.5 mm, 1 mm, 0 mm, etc.

A minimum distance between a connection pin 131 of one of multiple parameter interfaces 130 and an opening edge 1141 of a corresponding opening 114 may be less than 3 mm, or minimum distances between connection pins 131 of all parameter interfaces 130 and opening edges 1141 of corresponding openings 114 may be all less than 3 mm. Of course, the latter can further reduce a size of the housing 110, so as to further miniaturize the monitoring auxiliary device 100.

In some embodiments, the monitoring auxiliary device 100 further includes an in-position control mechanism (not shown in the figures), wherein the in-position control mechanism is configured to detect a connection state between at least one parameter interface 130 and a parameter accessory, and control to power off the parameter interface 130, when said parameter interface 130 is not in electrical connection with the parameter accessory, thereby avoiding a problem of electric shock caused by an accidental touch of a patient on said parameter interface 130.

As shown in FIG. 8, the connection pin 131, which is configured to electrically connect the parameter interface 130 with the parameter accessory, includes at least one first pin 1311. Wherein the in-position control mechanism is configured to detect a connection state between the first pin 1311 of at least one parameter interface 130 and a corresponding parameter accessory, and control the first pin 1311 of the at least one parameter interface 130 to power off, when the first pin 1311 of the at least one parameter interface 130 is not in electrical connection with the corresponding parameter accessory.

It can be understood that when there are multiple first pins 1311, if the first pins 1311 of the parameter interface 130 are not in electrical connection with the corresponding parameter accessory, the in-position control mechanism may control to power off one first pin 1311, or control to power off multiple even all first pins 1311. The more first pins which are power-off, the smaller a risk of electric shock caused by an accidental touch of a patient on the parameter interface 130.

In some embodiments, the in-position control mechanism includes an in-position detection component and a control module, wherein the control module is arranged on the circuit board 120, at least one parameter interface 130 is correspondingly arranged with one in-position detection component, wherein the in-position detection component is configured to detect a state of electrical connection between a corresponding parameter interface 130 and a parameter accessory, and configured to output a corresponding first signal to the control module, when the corresponding parameter interface 130 is not in electrical connection with the parameter accessory; and the control module is configured to control, according to the first signal, to power off at least one first pin 1311 of the parameter interface 130 that corresponds to the in-position detection component.

There are multiple manners for detecting whether the parameter interface 130 is in electrical connection with the parameter accessory through the in-position detection component. For example, whether the parameter interface 130 is in electrical connection with the parameter accessory may be determined by detecting a potential of at least one first pin 1311 of the parameter interface 130. Specifically, the in-position detection component is configured to detect a potential of at least one first pin 1311 of the parameter interface 130 which corresponds to said in-position detection component, and to output a first signal to the control module, when the potential of the first pin 1311 is greater than or equal to a first potential threshold. Wherein, the in-position detection component is configured to set a corresponding connection pin 131 to the first potential threshold, and when a first pin 1311 of the parameter interface 130 which corresponds to the in-position detection component is in electrical connection with a corresponding parameter accessory, the first pin 1311 is at a second potential threshold, wherein the first potential threshold is greater than the second potential threshold.

In some embodiments, the connection pin 131 of the parameter interface 130 further includes at least one second pin 1312, wherein the first pin 1311 and the second pin 1312 are in respective electrical connection with different parameter modules 1213 within a same parameter area 1201, wherein the first pin 1311 and the second pin 1312 are in respective electrical connection with different parameter accessories. When the in-position detection component is configured to detect a potential of the at least one second pin 1312 of the parameter interface 130, which interfaces corresponds to the in-position detection component, the in-position detection component is configured to output a second signal to the control module, when the potential of said at least one second pin 1312 is greater than or equal to a third potential threshold, and the control module is configured to control, according to the second signal, said at least one second pin 1312 of the parameter interface 130, which interfaces corresponds to the in-position detection component, to be powered off. When the second pin 1312 of the parameter interface 130 is in electrical connection with a corresponding parameter accessory, the potential of the second pin 1312 is less than or equal to a fourth potential threshold, wherein the third potential threshold is greater than the fourth potential threshold.

It can be understood that when the parameter interface 130 is in electrical connection with two parameter accessories at the same time, the parameter interface 130 is generally connected with a connector 210, and then connected with the two parameter accessories in one-to-one correspondence through two connection wires, which extend from the connector 210. When only one connection wire is in electrical connection with the parameter accessory, and the other connection wire is not in electrical connection with the parameter accessory, an end of the other connection wire to be in electrical connection with a parameter accessory, is kept in a power-off state, so as to prevent a patient from mistakenly touching said end of the other connection wire to cause an electric shock.

In this embodiment of this disclosure, when the first pin 1311 and the second pin 1312 in electrical connection with different parameter accessories are not in electrical connection with corresponding parameter accessories, the first pin 1311 and the second pin 1312 can be respectively kept in a power-off state under a control of the control module, so as to effectively avoid a problem of electric shock, which is caused when the parameter interface 130 is in electrical connection with a corresponding parameter accessory through one of the first pin 1311 and the second pin 1312 via one connection wire, while the other one of the first pin 1311 and the second pin 131 contacts with a patient through the other connection wire.

In some embodiments, the in-position detection component includes a sensor switch, wherein the sensor switch is triggered, when a corresponding parameter interface 130 is in electrical connection with a parametric accessory. The sensor switch may be arranged at the housing 110, or may be arranged at the parameter interface 130 or other positions that can be triggered by the parameter accessory. The sensor switch includes a micro switch or a photoelectric switch.

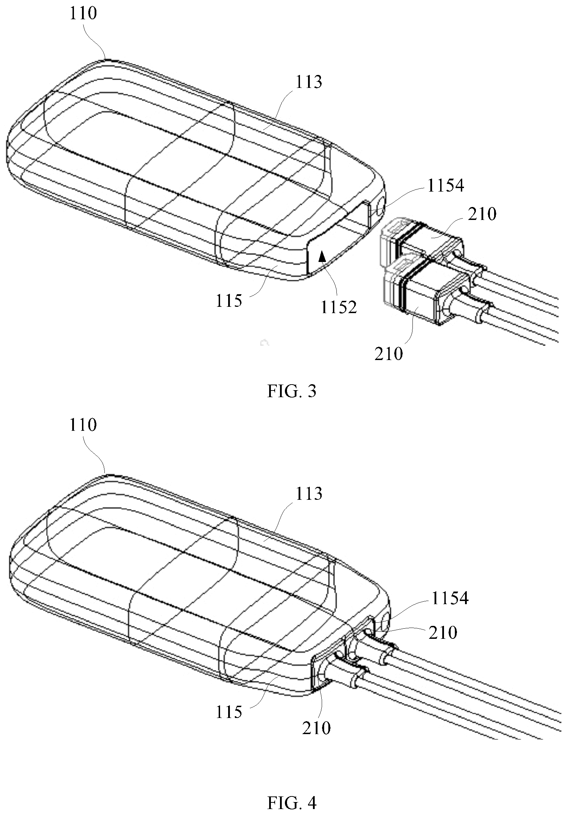

As shown in FIGS. 3 to 5, an outer surface 113 of the housing 110 is arranged with multiple holes 114 which respectively extend to an inner surface 111 and are connected with a mounting cavity 112, wherein each opening 114 enables at least one parameter interface 130 to be in electrical connection with an external parameter accessory. The parameter accessory is in electrical connection with the connector 210, and the connector 210 is in electrical connection with the parameter interface 130, so as to enable an electrical connection between the parameter accessory and the parameter interface 130.

The housing 110 further includes a surrounding portion 115, which surrounds at least a portion of at least one connector 210, when the connector 210 is in electrical connection with the parameter interface 130, so as to make a gap between the connector 210 and the housing 110 smaller, or make a gap between two adjacent connectors 210 smaller, for facilitating disinfection of the monitoring auxiliary device 100.

In some embodiments, the surrounding portion 115 encloses an accommodating groove 1152 that is connected with one end of at least one opening 114, which end is away from the mounting cavity 112. When the connector 210 is in electrical connection with the parameter interface 130, which corresponds to the opening 114 that is connected with the accommodating groove 1152, at least a portion of the connector 210 is located inside the accommodating groove 1152, so as to enable the surrounding portion 115 to surround at least a portion of the connector 210.

The accommodating groove 1152 may be connected with respective ends of multiple openings 114, which ends are away from the mounting cavity 112, a first mounting opening 1154 is configured to enable multiple connectors 210 to be inserted into the accommodating groove 1152. Therefore, the multiple connectors 210 can be inserted into the accommodating groove 1152 and in electrical connection with corresponding parameter interface(s) 130, so that the surrounding portion 115 can surround the multiple connectors 210 at the same time, so as to further improve disinfection convenience of the monitoring auxiliary device 100.

Certainly, the accommodating groove 1152 of the surrounding portion 115 can be only connected with one opening 114, when the connector 210 is inserted into the accommodating groove 1152 of the surrounding portion 115, the surrounding portion 115 only surrounds one connector 210. For multiple openings 114 of the housing 110, one surrounding portion 115 can be respectively arranged for each corresponding opening, or only some openings are arranged with a surrounding portion 115. Of course, the former can surround each connector 210, so as to further improve disinfection convenience of the monitoring auxiliary device 100.

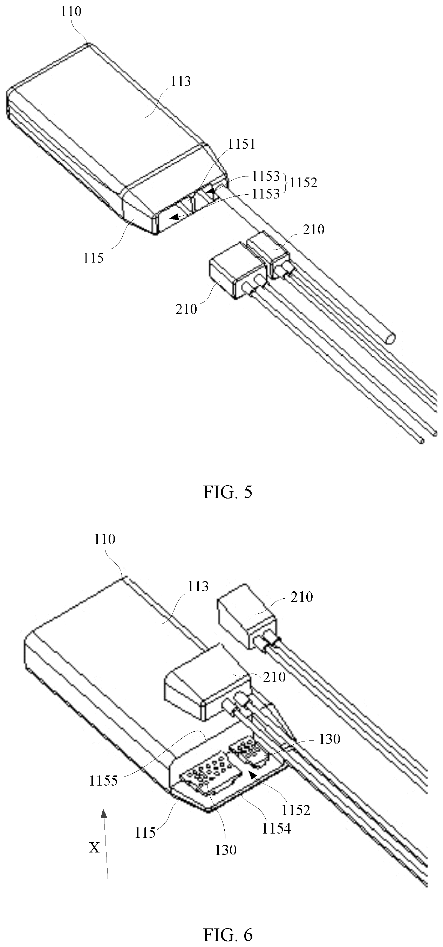

In an embodiment of this disclosure, there are multiple manners in which the connector 210 is inserted into the accommodating groove 1152 and connected with the parameter interface 130. For example, as shown in FIGS. 3 and 5, the accommodating groove 1152 includes a first mounting opening 1154 located at one end of the surrounding portion 115, which end is away from the opening 114, wherein the accommodating groove 1152 is inserted into the first mounting opening 1154 and in electrical connection with the parameter interface 130. The surrounding portion 115 is a closed structure along a circumferential direction of the accommodating groove 1152. Therefore, when the connector 210 is inserted into the accommodating groove 1152 through the first mounting opening 1154 and in electrical connection with the parameter interface 130, the surrounding portion 115 can surround a periphery of the connector 210, so as to further improve disinfection convenience of the monitoring auxiliary device 100.

Alternatively, as shown in FIG. 6, the accommodating groove 1152 further includes a second mounting opening 1155, which is located on a side of the surrounding portion 115 along a first direction X, wherein an angel forms between the first direction X and a direction from an opening 114 to a first mounting opening 1154, wherein the connector 210 is inserted into the accommodating groove 1152 through the second mounting opening 1155, so as to enable the connector 210 to be in electrical connection with a parameter interface 130 that corresponds to the accommodating groove 1152.

In some embodiments, as shown in FIG. 5, the housing 110 includes at least one dividing plate 1151 located inside the accommodation groove 1152, the dividing plate 1151 divides the accommodation groove 1152 into multiple sub-grooves 1153, which are in respective connection with the first mounting opening 1154, wherein each sub-groove 1153 is connected with one end of the at least one opening 114, which end is away from the mounting cavity 112, and each sub-groove 1153 is used for accommodating at least one connector 210. When multiple connectors 210 are respectively inserted into the multiple sub-grooves 1153 of the accommodating groove 1152, the multiple connectors 210 are in electrical connection with corresponding parameter interface(s) 130. Moreover, the dividing plate 1151 can separate parameter interfaces 130 corresponding to two adjacent sub-grooves 1153, so as to further improve an insulation effect between the parameter interfaces 130.

In some embodiments, the monitoring auxiliary device 100 is connected with a support mounted on a bedrail, so as to mount the monitoring auxiliary device 100 on the bedrail. Specifically, a support is mounted on a bedrail of a patient bed, and the monitoring auxiliary device 100 is detachably connected with the bracket.

An embodiment of this disclosure further provides a parameter connection component. The parameter connection component is configured to electrically connect a parameter accessory with a monitoring auxiliary device, so as to implement data transmission between the parameter accessory and the monitoring auxiliary device. The monitoring auxiliary device 100 includes a parameter interface 130 and at least two parameter modules 1213 in electrical connection with the parameter interface 130, wherein the at least two parameter modules 1213 are configured to acquire physiological parameters. Structures of the parameter interface 130, the parameter module 1213, and other components of the monitoring auxiliary device 100 may refer to above embodiments. Of course, the parameter interface 130 and the parameter modules 1213 of the monitoring auxiliary device 100 may also be embodiments other than the above monitoring auxiliary device 100, and only if the monitoring auxiliary device 100 have at least two parameter modules 1213 in electrical connection with one same parameter interface 130.

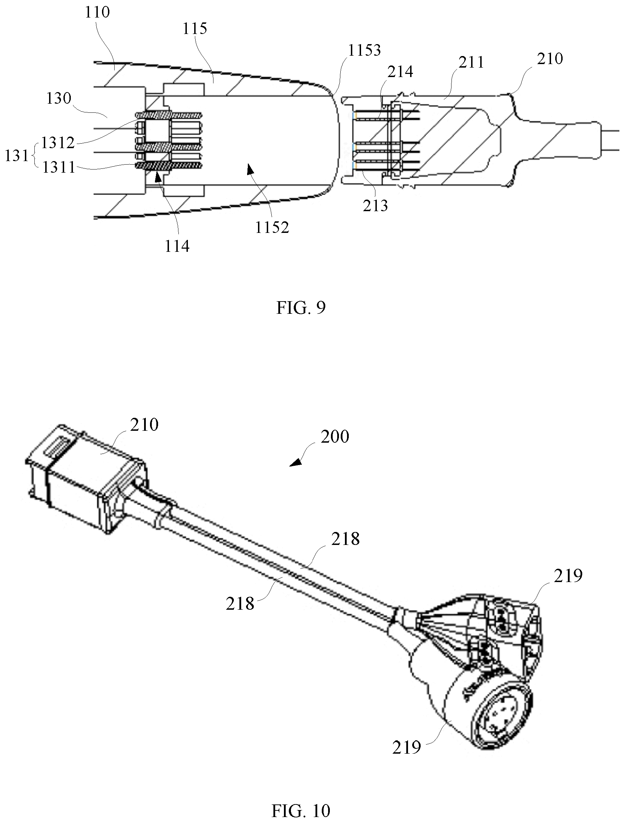

In some embodiments, as shown in FIG. 10, the parameter connection member 200 includes a connector 210, and at least two sub-connectors 219 in electrical connection with the connector 210, wherein each sub-connector 219 is in electrical connection with at least one parameter accessory. The sub-connector 219 may always remain in electrical connection with a corresponding parameter accessory, i.e., the sub-connector 219 is in connection with the corresponding parameter accessory, which connection is not capable of being disconnected. Alternatively, the sub-connector 219 can also be in connection with the corresponding parameter accessory, which connection is capable of being disconnected, such as by means of insertion, clamping, etc.

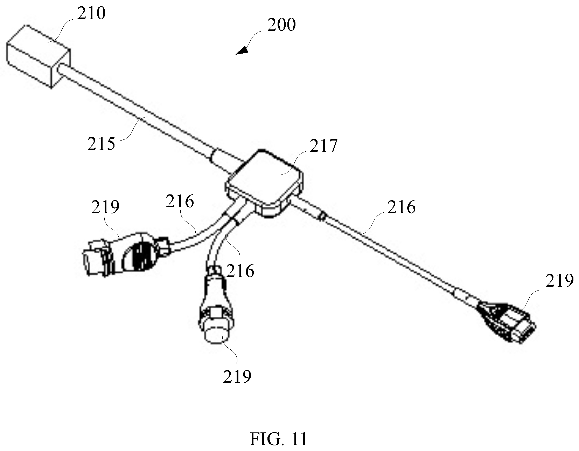

Each sub-connector 219 of the parameter connection component 200 may be in respective electrical connection with the connector 210 by means of different connection wiring harnesses 218. Alternatively, as shown in FIG. 11, the parameter connection component 200 includes a wiring harness 215 and multiple wiring sub-harnesses 216, one end of the wiring harness 215 is connected with the connector 210, each sub-connector 219 is respectively connected with the other end of the wiring harness 215 by a wiring sub-harness 216. A wire adapter 217 may be connected at the other end of the wiring harness 215, and the other end of the wiring harness 215 is connected with each wiring sub-harness 216 by the wire adapter 217.

The connector 210 of the parameter connection component 200 is in electrical connection with the parameter interface 130 of the monitoring auxiliary device 100, so as to electrically connect at least two parameter accessories with at least two parameter modules 1213 in one-to-one correspondence. Therefore, one parameter interface 130 of the monitoring auxiliary device 100 may be in electrical connection with at least two parameter accessories by means of the parameter connection component 200, so as to enable the monitoring auxiliary device 100 to acquire at least two types of physiological parameters. In such a way, the number of parameter interface(s) 130 of the monitoring auxiliary device 100 can be reduced, while maintaining the number of physiological parameters monitored by the monitoring auxiliary device 100 unchanged or maintaining the number of external parameter accessories in electrical connection with the monitoring auxiliary device unchanged, thus miniaturizing the monitoring auxiliary device 100 more.

As shown in FIGS. 8 and 9, the parameter interface 130 includes at least one first pin 1311 and at least one second pin 1312, which are in respective electrical connection with different parameter modules 1213. In some embodiments, the connector 210 includes a first connection element 213 in electrical connection with a first pin 1311 of the parameter interface 130 and a second connection 214 in electrical connection with the second pin 1312 of the parameter interface 130, so as to electrically connect the connector 210 with two parameter modules 1213 of the monitoring auxiliary device 100. The first connection element 213 and the second connection element 214 of the connector 210 are in respective electrical connection with different sub-connectors, so as to enable two parameter accessories to be in respective electrical connection with two parameter modules 1213 of the monitoring auxiliary device 100 in one-to-one correspondence.

Of course, the parameter interface 130 may further include at least one third pin in electrical connection with a parameter module 1213, wherein parameter modules 1213 connected with the first pin 1311, the second pin 1312 and the third pin are different. Correspondingly, the connector 210 includes a third connection element in electrical connection with the sub-connector 219, and parameter accessories connected with the first connection element 213, the second connection element 214 and the third connector are different. The third connector is in electrical connection with the third pin of the parameter interface 130, so as to enable three parameter accessories to be in respective electrical connection with three parameter modules 1213 of the monitoring auxiliary device 100 in one-to-one correspondence.

It may be understood that the parameter interface 130 may further include a fourth pin and a fifth pin, which are in electrical connection with different parameter modules 1213, and this may be specifically determined according to the number of parameter modules 1213 connected with the parameter interface 130. Correspondingly, the connector 210 includes a fourth connection element, a fifth connection element and the likes, which are in electrical connection with different sub-connectors 219, wherein the fourth connection element is in electrical connection with the fourth pin of the parameter interface 130, and the fifth connection element is in electrical connection with the fifth pin of the parameter interface 130, so as to enable four or five parameter accessories to be in respective electrical connection with four or five parameter modules 1213 of the monitoring auxiliary device 100 in one-to-one correspondence.

In some embodiments, as shown in FIG. 9, the first connection element 213 is in connection with the first pin 1311 via plug-in. The first connection element 213 includes a pin (or a receptacle), the first pin 1311 includes a receptacle (or a pin), so as to enable a plugged connection between the first connection element 213 and the first pin 1311 by plugging the pin into the receptacle.

Specifically, the connector 210 includes a housing body 211, the first connection element 213 is a pin, which is fixedly mounted inside the housing body 211. The first pin 1311 of the parameter interface 130 includes a receptacle, and after the housing body 211 of the connector 210 is inserted into the accommodating groove 1152 of the housing 110, the pin of the connector 210 is inserted into the receptacle of the first pin 1311, so as to enable the first connection element 213 to be in electrical connection with the first pin 1311.

Alternatively, as shown in FIG. 8, the first connection element 213 includes a first elastic pin, wherein the first elastic pin abuts against the first pin 1311, so as to enable the first connection element 213 to be in electrical connection with the first pin 1311. Specifically, the connector 210 includes a housing body 211, the first elastic pin is mounted inside the housing body 211, wherein after the housing body 211 of the connector 210 is inserted into the accommodating groove 1152 of the housing 110, a head of the first elastic pin abuts against the first pin 1311, so as to enable the first connection element 213 to be in electrical connection with the first pin 1311.

Similarly, the second connection element 214 is for plugged connection with the second pin 1312. The second connection element 214 includes a pin (or a receptacle), the second pin 1312 includes a receptacle (or a pin), so as to enable a plugged connection between the second connection element 214 and the second pin 1312 by plugging the pin into the receptacle.

Specifically, the connector 210 includes a housing body 211, the second connection element 214 is a pin, and the pin is fixedly mounted inside the housing body 211. The second pin 1312 of the parameter interface 130 includes a receptacle, wherein after the housing body 211 of the connector 210 is inserted into the accommodating grove 1152 of the housing 110, the pin of the connector 210 is inserted into the receptacle of the second pin 1312, so as to enable a plugged connection between the second connection element 214 and the second pin 1312.

Alternatively, as show in FIG. 8, the second connection element 214 includes a second elastic pin, wherein the second elastic pin abuts against the second pin 1312, so as to enable the second connection element 214 to be in electrical connection with the second pin 1312. Specifically, the connector 210 includes a housing body 211, the second elastic pin is mounted inside the housing body 211,wherein after the housing body 211 of the connector 210 is inserted into the accommodating grove 1152 of the housing 110, a head of the second elastic pin abuts against the second pin 1312, so as to enable the second connection element 214 to be in electrical connection with the second pin 1312.

As shown in FIG. 7, the monitoring auxiliary device 100 includes a housing 110, which includes an inner surface 111 and an outer surface 113, wherein the inner surface 111 encloses a mounting cavity 112, at least a portion of the parameter interface 130 is mounted inside the mounting cavity 112, the outer surface 113 of the housing 110 is formed with at least one protrusion 116, which is provided with at least one opening 114 that extends to the inner surface 111 and is connected with the mounting cavity 112, wherein each opening 114 exposes at least one parameter interface 130.

In some embodiments, the connector 210 of the parameter connection component 200 includes a housing body 211, wherein the housing body 211 includes a shielding portion 212, wherein the shielding portion 212 is formed with a protection groove (unshown). When the connector 210 is connected with a corresponding parameter interface 130, a protrusion 116 that corresponds to the parameter interface 130 is at least partially located inside the protection groove. Thereof, the shielding portion 212 of the housing body 211 can surround a periphery of the protrusion 116, reduce a gap between the connector 210 and the housing 110, or reduce a gap between two adjacent connectors 210, thereby improving disinfection convenience of the monitoring auxiliary device 100.

An embodiment of this disclosure further provides a circuit board structure, which includes a circuit board and two or more parameter interfaces, wherein specific structures of the circuit board and the parameter interface may refer to above embodiments. Since the structure of the circuit board adopts all technical solutions of all of above embodiments, at least all benefits brought by the technical solutions of the above embodiments are provided, which is not repeated.

In some embodiments, the circuit board 120 includes multiple parameter areas 1201 that are electrically isolated from each other, at least one parameter module 1213 is arranged within each parameter area 1201, wherein at least two parameter modules 1213 are arranged within at least one parameter area 1201, wherein the parameter modules 1213 are configured to acquire physiological parameters. A parameter interface 130 is in electrical connection with at least one parameter module 1213, the number of parameter interface(s) 130 is less than the number of parameter modules 1213, wherein each parameter interface 130 is in electrical connection with at least one external parameter accessory.

The circuit board structure provided in embodiments of this disclosure arranges at least two parameter modules 1213 inside at least one parameter area 1201 of the circuit board, and enables parameter modules 1213 inside the parameter area 1201 to be in electrical connection with corresponding parameter interface(s) 130, wherein the number of parameter interface(s) 130 is less than a corresponding number of parameter modules 1213, so as to enable at least two parameter modules 1213 to be in electrical connection with one parameter interface 130 and further in respective electrical connection with external parameter accessories through said one parameter interface 130. In such a way, the number of parameter interface(s) 130 of the monitoring auxiliary device can be reduced, while maintaining the number of physiological parameters monitored by the monitoring auxiliary device 100 unchanged or maintaining the number of external parameter accessories in electrical connection with the monitoring auxiliary device 100 unchanged, thus miniaturizing the monitoring auxiliary device 100 more.

In particular, when the parameter areas 1201 of the circuit board 120 are electrically isolated from each other, the parameter interface 130 can satisfy a safety requirement without providing a shielding structure, which can further reduce a size of the parameter interface 130 and satisfy miniaturization requirement of a monitoring interface to the greatest extent.

The parameter module 1213 inside at least one parameter area 1201 is in electrical connection with a same parameter interface 130. In additional, a connection pin 131 of the parameter interface 130 being in electrical connection with a parameter accessory, includes at least one first pin 1311. The monitoring auxiliary device 100 further includes an in-position control mechanism, which is configured to detect a state of electrical connection between the at least one first pin 1311 of at least one parameter interface 130 and a corresponding parameter accessory, and control the at least one first pin 1311 of the control parameter interface 130 to power off, when the at least one first pin 1311 of the parameter interface 130 is not in electrical connection with the corresponding parameter accessory. The structures of the circuit board 120 and the parameter interface 130 may refer to any one of the above embodiments, which is not repeated.

An embodiment of this disclosure further provides a monitoring auxiliary device, which includes a housing, a circuit board, and two or more parameter interfaces, wherein specific structures of the housing, the circuit board, and the parameter interface may refer to above embodiments. Since the monitoring auxiliary device adopts all technical solutions of all above embodiments, at least all benefits brought by the technical solutions of the above embodiments are provided, which is not repeated.

The monitoring auxiliary device 100 includes a housing 110, a circuit board 120 and a parameter interface 130, wherein the housing 110 includes an inner surface 111 and an outer surface 113, wherein the inner surface 111 encloses a mounting cavity 112, the circuit board 120 is mounted inside the mounting cavity 112 and includes two or more parameter areas 1201. At least one parameter module 1213 is arranged inside each parameter area 1201, wherein the parameter module 1213 is configured to acquire a physiological parameter. A parameter interface 130 is in electrical connection with at least one parameter module 1213, the number of parameter interface 130 is less than the number of parameter modules 1213, wherein each parameter interface 130 is in electrical connection with at least one external parameter accessory.

The monitoring auxiliary device 100 provided in embodiments of this disclosure, arranges at least two parameter modules 1213 inside at least one parameter area 1201 of a circuit board 120, so as to enable the parameter modules 1213 inside the parameter area(s) 1201 to be in electrical connection with a corresponding parameter interface 130, wherein the number of parameter interface(s) 130 is less than the number of parameter modules 1213, such that at least two parameter modules 1213 are in electrical connection with one parameter interface 130, and are in respective electrical connection with external parameter accessories parameter interface 130 through said one parameter interface. In such a way, the number of parameter interfaces 130 of the monitoring auxiliary device 100 can be reduced, while maintaining the number of physiological parameters monitored by the monitoring auxiliary device 100 unchanged or maintaining the number of external parameter accessories in electrical connection with the monitoring auxiliary device 100, thus miniaturizing the monitoring auxiliary device more.

In particular, when the parameter areas 1201 of the circuit board 120 are electrically isolated from each other, the parameter interface 130 can satisfy a safety requirement without providing a shielding structure, which can further reduce a size of the parameter interface 130 and satisfy a miniaturization requirement of a monitoring interface to the greatest extent.

Wherein, parameter modules 1213 within each parameter area 1201 are in electrical connection with one same parameter interface 130.

An outer surface 113 of the housing 110 is arranged with multiple openings 114, which respectively extend to an inner surface 111 and connect with the mounting cavity 112, wherein each opening 114 enables at least one parameter interface 130 to be in electrical connection with an external parameter accessory. Wherein each opening 114 includes an opening edge 1141 located on the outer surface 113, the parameter interface 130 includes a connection pin 131 in electrical connection with a parameter accessory, wherein the connection pin 131 is located inside the mounting cavity 112 and/or the opening 114, a minimum distance between the connection pin 131 of at least one parameter interface 130 and the opening edge 1141 of a corresponding opening 114 is less than 3 mm.

In addition, the connection pin 131 of the parameter interface 130, which pin is in electrical connection with the parameter accessory, includes at least one first pin 1311. The monitoring auxiliary device 100 further includes an in-position control mechanism, which is configured to detect a state of electrical connection between at least one first pin 1311 of at least one parameter interface 130 and a corresponding parameter accessory, and control the at least one first pin 1311 of the at least one parameter interface 130 to power off, when the at least one first pin 1311 of the parameter interface 130 is not in electrical connection with the corresponding parameter accessory.

The structures of the housing 110, the circuit board 120 and the parameter interface 130 may refer to the above embodiments, which is not repeated here.

An embodiment of this disclosure further provides a medical device, which includes a housing, a circuit board, and two or more parameter interfaces. Specific structures of the housing, the circuit board, and the parameter interface may refer to above embodiments. Since the medical device adopts all technical solutions of all above embodiments, at least all benefits brought by the technical solutions of above embodiments are provided, which is not repeated here.

In this embodiment of this disclosure, the medical device may be a monitoring auxiliary device, or may be another medical device that can be in electrical connection with multiple parameter accessories.

In some embodiments, the housing 110 of the medical device includes an inner surface 111 and an outer surface 113, wherein the inner surface 111 encloses a mounting cavity 112, the circuit board 120 is mounted inside the mounting cavity 112 and includes multiple parameter areas 1201 that are electrically isolated from each other, wherein at least one parameter module 1213 is arranged within each parameter area 1201, and at least one parameter area 1201 is arranged with at least two parameter modules 1213 for acquiring physiological parameters. The parameter interface 130 is in electrical connection with at least one parameter module 1213, the number of parameter interface(s) 130 is less than the number of parameter modules 1213, wherein each parameter interface 130 is in electrical connection with at least one external parameter accessory.

Wherein the parameter modules 1213 within at least one parameter area 1201 are in electrical connection with one same parameter interface 130. An outer surface 113 of the housing 110 is arranged with multiple openings 114, which respectively extend to the inner surface 111 and connect with the mounting cavity 112, wherein each opening 114 enables at least one parameter interface 130 to be in electrical connection with an external parameter accessory. Wherein each opening 114 includes an opening edge 1141 located on the outer surface 113, the parameter interface 130 includes a connection pin 131 in electrical connection with a parameter accessory, wherein the connection pin 131 is located inside the mounting cavity 112 and/or the opening 114, a minimum distance between the connection pin 131 of at least one parameter interface 130 and the opening edge 1141 of a corresponding opening 114 is less than 3 mm.

The connection pin 131 of the parameter interface 130, which pin is in electrical connection with the parameter accessory, includes at least one first pin 1311. The monitoring auxiliary device 100 further includes an in-position control mechanism, which is configured to detect a state of electrical connection between at least one first pin 1311 of at least one parameter interface 130 and a corresponding parameter accessory, and control the at least one first pin 1311 of the at least one parameter interface 130 to power off, when the at least one first pin 1311 of the parameter interface 130 is not in electrical connection with the corresponding parameter accessory.

The structures of the housing 110, the circuit board 120 and the parameter interface 130 may refer to the above embodiments, which is not repeated here.

An embodiment of this disclosure further provides a parameter acquisition assembly, which includes a connector 210 and at least two parameter accessories of different types of physiological parameters, each parameter accessory is in electrical connection with the connector 210, and the connector 210 is in electrical connection with a parameter interface 130 of a monitoring auxiliary device, so as to transmit parameters acquired by the at least two parameter accessories to the monitoring auxiliary device. A specific structure of the parameter accessory may refer to above embodiments, a structure of the connector 210 may also refer to the above embodiments, which is not repeated here.

It should be noted that the connector 210 may be directly connected with the parameter accessory through a wired or wireless manner between the sub-connector and each parameter accessory.

In some embodiments, each parameter accessory may be in respective electrical connection with the connector 210 by different connection wiring harnesses 218. Specifically, the parameter acquisition assembly includes a connector 210, multiple parameter accessories and multiple connection wiring harnesses 218, the number of the multiple parameter accessories is equal to the number of the multiple connection wiring harnesses 218, respective ends of the multiple connection wiring harnesses 218 are in respective electrical connection with the multiple parameter accessories in one-to-one correspondence, and the other respective ends of the multiple connection wiring harnesses 218 are in electrical connection with the connector 210. The connection wiring harness 218 may be integrally formed with the corresponding parameter accessory, or the connection wiring harness 218 may be connected with the corresponding parameter accessory by welding and pasting.

In other embodiments, the parameter acquisition assembly may include a wiring harness 215 and multiple wiring sub-harnesses 216, one end of the wiring harness 215 is in electrical connection with the connector 210, and each parameter accessory is in respective electrical connection with the other end of the wiring harness 215 through the multiple wiring sub-harnesses 216. Specifically, the number of the multiple wiring sub-harnesses 216 of the parameter acquisition assembly is equal to the number of the multiple parameter accessories, respective ends of the multiple wiring sub-harnesses 216 are in respective electrical connection with the multiple parameter accessories in one-to-one correspondence, the other respective ends of the multiple wiring sub-harnesses 216 are in electrical connection with one end of the wiring harness 215, and the other end of the wiring harness 215 is in electrical connection with the connector 210. The wiring sub-harnesses 216 may be integrally formed with the corresponding parameter accessory, or the multiple wiring sub-harnesses 216 may be connected with the corresponding parameter accessory as a whole by welding, pasting, etc.

In some embodiments, the connector 210 includes a first connection element 213 and a second connection element 214, the first connection element 213 and the second connection element 214 are in respective electrical connection with different parameter accessories. The parameter interface 130 includes at least one first pin 1311 and at least one second pin 1312, which are in respective electrical connection with different parameter modules 1213, the first connection element 213 is in electrical connection with the first pin 1311, and the second connection element 214 is in electrical connection with the second pin 1312, so as to electrically connect the two parameter accessories with two parameter modules 1213 in one-to-one correspondence.

In some embodiments, the monitoring auxiliary device 100 includes a housing 110, which includes an inner surface 111 and an outer surface 113, wherein the inner surface 111 encloses a mounting cavity 112, at least a portion of the parameter interface 130 is mounted within the mounting cavity 112, the outer surface 111 of the housing 110 is formed with at least one protrusion 116, wherein the at least one protrusion 116 is provided with at least one opening 114 that extends to the inner surface 111 and is connected with the mounting cavity 112, wherein each opening 114 enables at least one parameter interface 130 to be in electrical connection with an external parameter accessory. The connector 210 includes a housing body 211, and the housing body 211 includes a shielding portion 212, which is formed with a protection groove. When the connector 210 is connected with the corresponding parameter interface 130, a protrusion 116 that corresponds to the parameter interface 130 is at least partially located inside the protection groove.

In the above embodiments, description of each embodiment has its own emphasis, and for that are not described in detail in a certain embodiment, reference may be made to relevant description of other embodiments.

The parameter connection component, the circuit board structure, the parameter acquisition assembly, the monitoring auxiliary device, and the medical device provided inside the embodiments of this disclosure have been described in detail above, and specific examples are used herein to describe principles and embodiments of this disclosure, and description of above embodiments is only used to help understand technical solutions of this disclosure and a core idea thereof. It should be understood by those skilled in the art that technical solutions described in above embodiments may still be modified, or some technical features may be equivalently replaced; and these modifications or replacements do not make the essence of corresponding technical solutions depart from the scope of the technical solutions of the embodiments of this disclosure.

## Claims

1. A monitoring auxiliary device, **characterized in that**, comprising:
a housing, which comprises an inner surface and an outer surface, wherein the inner surface encloses a mounting cavity;
a circuit board, which is mounted inside the mounting cavity, wherein the circuit board comprises multiple parameter areas that are electrically isolated from each other; wherein at least one parameter module is arranged inside each parameter area of the multiple parameter areas; at least two parameter modules are arranged inside at least one parameter area of the multiple parameter areas; wherein each parameter module is configured to acquire a physiological parameter;
multiple parameter interfaces, wherein each parameter area of the multiple parameter areas is correspondingly connected with at least one parameter interface of the multiple parameter interfaces, each parameter module is in electrical connection with at least one parameter interface of the multiple parameter interfaces; wherein a number of parameter interface(s), which is(are) connected with at least one parameter area, is less than a number of parameter modules that are arranged inside said at least one parameter area, wherein at least two parameter modules are arranged inside said at least one parameter area; wherein each parameter area of the multiple parameter areas is capable of being in electrical connection with at least one external parameter accessory; and
a data output interface, which is capable of being connected with a monitor through a cable, so as to output to the monitor, in a wired manner, the acquired physiological parameters and/or data that are generated based on processing the acquired physiological parameters.

2. The monitoring auxiliary device according to claim 1, **characterized in that**, at least two parameter modules inside at least one parameter area of the multiple parameter areas are in electrical connection with one parameter interface, wherein said at least two parameter modules are arranged inside said at least one parameter area.

3. The monitoring auxiliary device according to claim 2, **characterized in that**, at least two parameter modules are arranged inside each parameter area of the multiple parameter areas, said at least two parameter modules are in respective electrical connection with one corresponding parameter interface.

4. The monitoring auxiliary device according to claim 1, **characterized in that**, the monitoring auxiliary device is a wearable device; and/or
the monitoring auxiliary device further comprises a wireless communication module that is in electrical connection with the circuit board, wherein the wireless communication module wirelessly outputs to the monitor the acquired physiological parameters and/or the data that are generated based on processing the acquired physiological parameters.

5. The monitoring auxiliary device according to claim 1, **characterized in that**, an insulated band is arranged between two adjacent parameter areas of the multiple parameter areas, so as to enable said two adjacent parameter areas to be electrically isolated.

6. The monitoring auxiliary device according to claim 1, **characterized in that**, a minimum gap between parameter modules that are respectively located inside two adjacent parameter areas of the multiple parameter areas is greater than or equal to 5.2 mm.

7. The monitoring auxiliary device according to claim 1, **characterized in that**, the multiple parameter areas are independently grounded to be electrically isolated from each other.

8. The monitoring auxiliary device according to claim 1, **characterized in that**, an isolated communication module and/or an isolated power supply module are/is arranged between at least two adjacent parameter areas of the multiple parameter areas.

9. The monitoring auxiliary device according to claim 1, **characterized in that**, the circuit board further comprises a non-parameter area that is adjacent to at least one parameter area of the multiple parameter areas, wherein an isolated communication module and/or an isolated power supply module are/is arranged between the non-parameter area and said at least one parameter area that is adjacent to the non-parameter area.

10. The monitoring auxiliary device according to claim 9, **characterized in that**, each parameter area of the multiple parameter areas is adjacent to the non-parameter area; wherein the isolated communication module and/or the isolated power supply module are/is respectively arranged between the non-parameter area and said each parameter area.

11. The monitoring auxiliary device according to claim 9, **characterized in that**, the parameter module(s) inside said at least one parameter area that is adjacent to the non-parameter area is(are) in electrical connection with the isolated communication module and/or with the isolated power supply module that are/is arranged between said at least one parameter area and the non-parameter area.

12. The monitoring auxiliary device according to claim 9, **characterized in that**, the non-parameter area is arranged with a data processing module; wherein the isolated communication module is configured to transmit to the data processing module the physiological parameter(s) acquired by the parameter module(s) inside said at least one parameter area or the data generated based on processing said physiological parameter(s), the isolated power supply module is configured to perform power conversion between the non-parameter area and said at least one parameter area.

13. The monitoring auxiliary device according to claim 9, **characterized in that**, the non-parameter area is further arranged with one or more of: a data processing module, a pressure sensor, a power management module, a wireless communication module, a battery interface and a charging interface.

14. The monitoring auxiliary device according to any one of claims 1-13, **characterized in that**, at least one parameter area of the multiple parameter areas is arranged with a plug-in module, which is in electrical connection with the parameter module(s) inside the at least one parameter area, wherein at least one parameter interface of the multiple parameter interfaces is plugged into the plug-in module; and/or
at least one parameter interface of the multiple parameter interfaces is mounted inside a corresponding parameter area; and/or
the parameter module(s) inside at least one parameter area of the multiple parameter areas is(are) in electrical connection with corresponding parameter interface(s) through connection wire(s).

15. The monitoring auxiliary device according to any one of claims 1-13, **characterized in that**, the outer surface is arranged with multiple openings, which respectively extend to the inner surface and are connected with the mounting cavity, wherein each opening of the multiple openings enables at least one parameter interface of the multiple parameter interfaces to be in electrical connection with the at least one external parameter accessory;
wherein an opening of the multiple openings comprises an opening edge located on the outer surface, the at least one parameter interface comprises a connection pin that is in electrical connection with the at least one external parameter accessory, wherein the connection pin is located inside the mounting cavity and/or said opening, wherein a minimum distance between the connection pin of the at least one parameter interface and the opening edge of the opening is less than 3 mm.

16. The monitoring auxiliary device according to any one of claims 1-13, **characterized in that**, a connection pin of at least one parameter interface of the multiple parameter interfaces comprises at least one first pin, wherein the connection pin is in electrical connection with the at least one external parameter accessory;
the monitoring auxiliary device further comprises an in-position control mechanism, which is configured to detect a state of electrical connection between the at least one first pin of the at least one parameter interface and a corresponding external parameter accessory, and configured to control the at least one first pin of the at least one parameter interface to be powered off, when the at least one first pin of the at least one parameter interface is not in electrical connection with the corresponding external parameter accessory.

17. The monitoring auxiliary device according to claim 16, **characterized in that**, the in-position control mechanism comprises an in-position detection component and a control module, wherein the control module is arranged on the circuit board, at least one parameter interface of the multiple parameter interfaces is correspondingly arranged with one in-position detection component; wherein the in-position detection component is configured to detect a state of electrical connection between a corresponding parameter interface and a corresponding external parameter accessory, and configured to output a corresponding first signal to the control module, when the corresponding parameter interface is not in electrical connection with the corresponding external parameter accessory; the control module is configured to control, according to the first signal, the at least one first pin of the corresponding parameter interface that corresponds to the in-position detection component to be powered off.

18. The monitoring auxiliary device according to claim 17, **characterized in that**, the in-position detection component is configured to detect a potential of the at least one first pin of the corresponding parameter interface that corresponds to the in-position detection component, and configured to output the first signal to the control module, when the potential of the at least one first pin is greater than or equal to a first potential threshold;
wherein, when the at least one first pin of the corresponding parameter interface is in electrical connection with the corresponding external parameter accessory, the potential of the at least one first pin is less than or equal to a second potential threshold, wherein the first potential threshold is greater than the second potential threshold.

19. The monitoring auxiliary device according to claim 16, **characterized in that**, the connection pin further comprises at least one second pin, wherein the at least one first pin and the at least one second pin are in respective electrical connection with different parameter modules inside a same parameter area, wherein the at least one first pin and the at least one second pin are in respective electrical connection with different external parameter accessories;
the in-position detection component is configured to detect a potential of the at least one second pin of a corresponding parameter interface that corresponds to the in-position detection component, and configured to output a second signal to the control module, when the potential of the at least one second pin is greater than or equal to a third potential threshold; the control module is configured to control, according to the second signal, the at least one second pin of the corresponding parameter interface that corresponds to the in-position detection component to be powered off;
wherein, when the at least one second pin of the corresponding parameter interface is in electrical connection with a corresponding external parameter accessory, the potential of the at least one second pin is less than or equal to a fourth potential threshold, wherein the third potential threshold is greater than the fourth potential threshold.

20. The monitoring auxiliary device according to claim 17, **characterized in that**, the in-position detection component comprises a sensor switch, which is capable of being triggered, when the corresponding parameter interface is in electrical connection with the corresponding external parameter accessory.

21. The monitoring auxiliary device according to claim 20, **characterized in that**, the sensor switch comprises a micro switch or a photoelectric switch.

22. The monitoring auxiliary device according to any one of claims 1-13, **characterized in that**, the parameter modules comprise a measurement module for a body temperature and a measurement module for an invasive blood pressure, wherein the measurement module for a body temperature and the measurement module for an invasive blood pressure are arranged inside a same parameter area; or
the parameter modules comprise a measurement module for electrocardiogram and a measurement module for blood oxygen, wherein the measurement module for and the measurement module for blood oxygen are arranged inside a same parameter area.

23. The monitoring auxiliary device according to any one of claims 1-13, **characterized in that**, the outer surface is arranged with multiple openings, which respectively extend to the inner surface and are connected with the mounting cavity, wherein each opening of the multiple openings enables at least one parameter interface of the multiple parameter interfaces to be in electrical connection with the at least one external parameter accessory;
the housing further comprises a surrounding portion, which surrounds an accommodating groove, wherein the accommodating groove is connected with one end of at least one opening of the multiple openings, which end is away from the mounting cavity;
the accommodating groove comprises a first mounting opening that is located at one end of the surrounding portion, which end is away from the at least one opening, wherein the first mounting opening is configured to enable connector(s) that is(are) connected with the at least one parameter interface to be inserted into the accommodating groove and in electrical connection with the at least one parameter interface.

24. The monitoring auxiliary device according to claim 23, **characterized in that**, the accommodating groove is connected with respective ends of the multiple openings, which ends are away from the mounting cavity, wherein the first mounting opening is configured to enable multiple connectors to be inserted into the accommodating groove.

25. The monitoring auxiliary device according to claim 24, **characterized in that**, the housing further comprises at least one dividing plate that is located inside the accommodating groove, wherein the dividing plate divides the accommodating groove into multiple sub-grooves, which are respectively connected with the first mounting opening, wherein each sub-groove of the multiple sub-grooves is connected with one end of at least one opening of the multiple openings, which end is away from the mounting cavity, and said each sub-groove is configured to accommodate at least one connector of the multiple connectors.

26. The monitoring auxiliary device according to claim 23, **characterized in that**, the surrounding portion is a closed structure along a circumferential direction of the accommodating groove; or
the accommodating groove further comprises a second mounting opening that is located on one side of the surrounding portion along a first direction, wherein the first direction has an included angle with a direction from the at least one opening to the first mounting opening, the second mounting opening is connected with a surface of the first mounting opening at the surrounding portion, and the second mounting opening is configured to enable the connector(s) to be inserted into the accommodating groove.

27. The monitoring auxiliary device according to claim 1, **characterized in that**, the monitoring auxiliary device is connected with a support mounted on a bedrail.

28. A monitoring auxiliary device, **characterized in that**, comprising:
a housing, which comprises an inner surface and an outer surface, wherein the inner surface encloses a mounting cavity;
a circuit board, which is mounted inside the mounting cavity, wherein the circuit board comprises two or more parameter areas, wherein at least one parameter module is arranged inside each parameter area of the two or more parameter areas; the at least one parameter module is configured to acquire a physiological parameter; and
two or more parameter interfaces, wherein each parameter interface of the two or more parameter interfaces is in electrical connection with the at least one parameter module, a number of parameter interfaces is less than a number of parameter modules, wherein each parameter interface of the two or more parameter interfaces is capable of being in electrical connection with at least one external parameter accessory.

29. The monitoring auxiliary device according to claim 28, **characterized in that**, parameter modules inside at least one parameter area of the two or more parameter areas are in electrical connection with a same parameter interface.

30. The monitoring auxiliary device according to claim 28, **characterized in that**, the outer surface is arranged with multiple openings, which respectively extend to the inner surface and are connected with the mounting cavity, wherein each opening of the multiple openings enables at least one parameter interface of the two or more parameter interfaces to be in electrical connection with the at least one external parameter accessory;
wherein an opening of the multiple openings comprises an opening edge located on the outer surface, the at least one parameter interface comprises a connection pin that is in electrical connection with the at least one external parameter accessory, wherein the connection pin is located inside the mounting cavity and/or said opening, wherein a minimum distance between the connection pin of the at least one parameter interface and the opening edge of a corresponding opening is less than 3 mm.

31. The monitoring auxiliary device according to claim 28, **characterized in that**, a connection pin of at least one parameter interface of the two or more parameter interfaces, which connection pin is in electrical connection with the at least one external parameter accessory, comprises at least one first pin;
the monitoring auxiliary device further comprises an in-position control mechanism, which is configured to detect a state of electrical connection between the at least one first pin of the at least one parameter interface and a corresponding external parameter accessory, and configured to control the at least one first pin of the at least one parameter interface to be powered off, when the at least one first pin of the at least one parameter interface is not in electrical connection with the corresponding external parameter accessory.

32. A circuit board structure, **characterized in that**, comprising:
a circuit board, which comprises two or more parameter areas, wherein at least one parameter module is arranged inside each parameter area of the two or more parameter areas; the at least one parameter module is configured to acquire a physiological parameter; and
two or more parameter interfaces, wherein each parameter interface of the two or more parameter interfaces is in electrical connection with the at least one parameter module, a number of parameter interfaces is less than a number of parameter modules, wherein each parameter interface of the two or more parameter interfaces is capable of being in electrical connection with at least one external parameter accessory.

33. The circuit board structure according to claim 32, **characterized in that**, parameter modules inside at least one parameter area of the two or more parameter areas are in electrical connection with a same parameter interface.

34. The circuit board structure according to claim 32, **characterized in that**, a connection pin of at least one parameter interface of the two or more parameter interfaces, which connection pin is in electrical connection with the at least one external parameter accessory, comprises at least one first pin;
the monitoring auxiliary device further comprises an in-position control mechanism, which is configured to detect a state of electrical connection between the at least one first pin of the at least one parameter interface and a corresponding external parameter accessory, and configured to control the at least one first pin of the at least one parameter interface to be powered off, when the at least one first pin of the at least one parameter interface is not in electrical connection with the corresponding external parameter accessory.

35. A medical device, **characterized in that**, comprising:
a housing, which comprises an inner surface and an outer surface, wherein the inner surface encloses a mounting cavity;
a circuit board, which comprises two or more parameter areas, wherein at least one parameter module is arranged inside each parameter area of the two or more parameter areas; the at least one parameter module is configured to acquire a physiological parameter; and
two or more parameter interfaces, wherein each parameter interface of the two or more parameter interfaces is in electrical connection with the at least one parameter module, a number of parameter interfaces is less than a number of parameter modules, wherein each parameter interface of the two or more parameter interfaces is capable of being in electrical connection with the at least one external parameter accessory.

36. The medical device according to claim 35, **characterized in that**, parameter modules inside at least one parameter area of the two or more parameter areas are in electrical connection with a same parameter interface.

37. The medical device according to claim 35, **characterized in that**, the outer surface is arranged with multiple openings, which respectively extend to the inner surface and are connected with the mounting cavity, wherein each opening of the multiple openings enables at least one parameter interface of the two or more parameter interfaces to be in electrical connection with the at least one external parameter accessory;
wherein an opening of the multiple openings comprises an opening edge located on the outer surface, the at least one parameter interface comprises a connection pin that is in electrical connection with the at least one external parameter accessory, wherein the connection pin is located inside the mounting cavity and/or said opening, wherein a minimum distance between the connection pin of the at least one parameter interface and the opening edge of a corresponding opening is less than 3 mm.

38. The medical device according to claim 35, **characterized in that**, a connection pin of at least one parameter interface of the two or more parameter interfaces, which connection pin is in electrical connection with the at least one external parameter accessory, comprises at least one first pin;
the monitoring auxiliary device further comprises an in-position control mechanism, which is configured to detect a state of electrical connection between the at least one first pin of the at least one parameter interface and a corresponding external parameter accessory, and configured to control the at least one first pin of the at least one parameter interface to be powered off, when the at least one first pin of the at least one parameter interface is not in electrical connection with the corresponding external parameter accessory.

39. A parameter connection component, which is configured to electrically connect at least two parameter accessories of different types of physiological parameters with a monitoring auxiliary device;
**characterized in that**, the parameter connection component comprises a connector and at least two sub-connectors that are in electrical connection with the connector; wherein each sub-connector of the at least two sub-connectors is in electrical connection with at least one parameter accessory of the at least two parameter accessories, the connector is in electrical connection with a parameter interface of the monitoring auxiliary device, so as to transmit the physiological parameters acquired by the at least two parameter accessories to the monitoring auxiliary device.

40. The parameter connection component according to claim 39, **characterized in that**, the connector comprises a first connection element and a second connection element, which are in respective electrical connection with different sub-connectors of the at least two sub-connectors;
the parameter interface comprises at least one first pin and at least one second pin, which are in respective electrical connection with different parameter modules, wherein the first connection element is in electrical connection with the at least one first pin, the second connection element is in electrical connection with the at least one second pin, so as to electrically connect two parameter accessories of the at least two parameter accessories with two parameter modules of the different parameter modules in one-to-one correspondence.

41. The parameter connection component according to claim 40, **characterized in that**, the first connection element is in connection with the at least one first pin via plug-in, or the first connection element comprises a first elastic pin that abuts against the at least one first pin; and/or
the second connection element is in connection with the at least one second pin via plug-in, or the second connection element comprises a second elastic pin that abuts against the at least one second pin.

42. The parameter connection component according to claim 39, **characterized in that**, each sub-connector of the at least two sub-connectors is in respective electrical connection with the connector through different connection wiring harnesses.

43. The parameter connection component according to claim 39, **characterized in that**, further comprising a wiring harness and multiple wiring sub-harnesses, wherein one end of the wiring harness is in electrical connection with the connector, and each sub-connector of the at least two sub-connectors is in respective electrical connection with the other end of the wiring harness through respective wiring sub-harness of the multiple wiring sub-harnesses.

44. The parameter connection component according to claim 39, **characterized in that**, the monitoring auxiliary device comprises a housing, which comprises an inner surface and an outer surface, wherein the inner surface encloses a mounting cavity;
at least a portion of the parameter interface is mounted inside the mounting cavity, at least one protrusion is formed on the outer surface of the housing, wherein the at least one protrusion is arranged with at least one opening, which extends to the inner surface and is connected with the mounting cavity; wherein each opening of the at least one opening enables at least one parameter interface to be in electrical connection with the at least two parameter accessories;
the connector comprises a housing body, which comprises a shielding portion, wherein the shielding portion is formed with a protection groove, wherein, when the connector is connected with a corresponding parameter interface, the at least one protrusion that corresponds to the corresponding parameter interface is at least partially located inside the protection groove.

45. A parameter acquisition assembly, **characterized in that**, comprising a connector and at least two parameter accessories of different types of physiological parameters, wherein each parameter accessory of the at least two parameter accessories is in respective electrical connection with the connector, the connector is further in electrical connection with a parameter interface of a monitoring auxiliary device, so as to transmit the physiological parameters acquired by the at least two parameter accessories to the monitoring auxiliary device.

46. The parameter acquisition assembly according to claim 45, **characterized in that**, the connector comprises a first connection element and a second connection element, which are in respective electrical connection with different parameter accessories of the at least two parameter accessories;
the parameter interface comprises at least one first pin and at least one second pin, which are in respective electrical connection with different parameter modules, wherein the first connection element is in electrical connection with the at least one first pin, the second connection element is in electrical connection with the at least one second pin, so as to electrically connect two parameter accessories of the at least two parameter accessories with two parameter modules of the different parameter modules in one-to-one correspondence.

47. The parameter acquisition assembly according to claim 45, **characterized in that**, each parameter accessory of the at least two parameter accessories is in respective electrical connection with the connector through different connection wiring harnesses; or
the parameter acquisition assembly further comprises a wiring harness and multiple wiring sub-harnesses, wherein one end of the wiring harness is in electrical connection with the connector, and each parameter accessory of the at least two parameter accessories is in respective electrical connection with the other end of the wiring harness through respective wiring sub-harness of the multiple wiring sub-harnesses.

48. The parameter acquisition assembly according to claim 45, **characterized in that**, the monitoring auxiliary device comprises a housing, which comprises an inner surface and an outer surface, wherein the inner surface encloses a mounting cavity;
at least a portion of the parameter interface is mounted inside the mounting cavity, at least one protrusion is formed on the outer surface of the housing, wherein the at least one protrusion is arranged with at least one opening, which extends to the inner surface and is connected with the mounting cavity; wherein each opening of the at least one opening enables at least one parameter interface to be in electrical connection with the at least two parameter accessories;
the connector comprises a housing body, which comprises a shielding portion, wherein the shielding portion is formed with a protection groove, wherein, when the connector is connected with a corresponding parameter interface, the at least one protrusion that corresponds to the corresponding parameter interface is at least partially located inside the protection groove.
